# EUROPEAN PATENT APPLICATION

(11) **EP 4 830 632 A1**
(43) Date of publication of application: **28.10.2026**
(21) Application number: 26185960.7
(22) Date of filing: 20.05.2025
(51) Int. Cl.: A61B 5/367

(54) **SYSTEMS AND METHODS FOR CARDIAC DIAGNOSTICS**

(30) Priority: 20.05.2024 DE 102024114031; 19.05.2025 EP 25177220
(62) Document number(s) of the earlier application(s) in accordance with Art. 76 EPC: 25730358.6 / 4 704 702
(71) Applicant: CorLector GmbH, 90480 Nürnberg (DE)
(72) Inventor: CHMELEVSKY, Mikhail, 90480 Nürnberg (DE); STRÖBEL, Jörg Peter, 90480 Nürnberg (DE); POTYAGAYLO, Danila, 90480 Nürnberg (DE); KHAMZIN, Sviatoslav, 90480 Nürnberg (DE); BAZHUTINA, Anastasia, 90480 Nürnberg (DE)
(74) Representative: Schweiger, Martin

(57) **Abstract**

A computer-implemented method for generating a cardiac activation map is provided. The method comprises receiving data for at least one cardiac cycle obtained from ECG data of a patient and determining at least one parameter representing cardiac activation based on the received data. The method further comprises obtaining from a database at least one activation map, wherein the obtained at least one activation map is selected from the database based on the determined at least one parameter. The method further comprise generating an initial activation map based on the at least one activation map obtained from the database and generating a final activation map by optimizing the initial activation map.

## Description

The present application generally relates to systems and methods for cardiac diagnostics, and more particularly to techniques for analyzing and interpreting cardiac data using computer-based algorithms.

Reference is made to the earlier patent applications DE102024114031.3 of 20 May 2024 and EP25177220.8 of 19 May 2025, the priorities of which are herewith claimed, and the contents of which are herein incorporated by reference.

Cardiovascular arrhythmia are the leading cause of death in the western world. Even though some mechanisms of disease progression are known, optimal treatment approaches are non-existent for complex arrhythmias such as atrial fibrillation or some non-inducible polymorphic ventricular tachycardias. This is due to the fact that genesis of these diseases is a complex interplay between genetic factors and life-style resulting in highly unpredictable cardiac substrate changes and abnormalities in the excitation patterns.

Cardiological care may be broadly divided into monitoring and preventive care before and after the procedure on the one hand and acute treatment on the other hand. The former in general is ensured by practitioners during regular check-ups and in acute cases. Treatment is usually delivered either in the form of identifying and acutely ablating arrhythmogenic areas in the heart in the catheter laboratory (cathlab) or/and implantable devices based on the patient's diagnosis and individual characteristics of rhythm conduction and cardiac anatomy.

Invasive systems monitoring electrical activity of the heart can provide only local information, which is not sufficient for diagnosis of complex non-repeatable patterns of cardiac arrhythmia. Surface-ECG allows qualitative estimation of global conduction patterns. However, this is without 3D coordinates, and lacks sufficient resolution on the local scale.

Anatomical maps of the cardiac myocardium are sometimes very useful per se, but do not deliver any information on the electrical functionality of the heart.

Existing solutions for creating 3D-electroanatomical maps or panoramic - i.e., multichamber epi- and endocardial - visualization of the electrical activity of the heart based on non-invasive mapping depend on the use of specific electrode vests or electrode stripes. The number of electrodes is in the range of 160-200 electrodes. The whole thorax of the patient is covered with electrodes. I.e., placement of e.g. defibrillation patches or other sensors/actors is difficult. Location of the electrodes has to be visualized by 3D imaging. A patient individual model of the heart has to be acquired by CT or MRI.

EP3157423A4 shows a method that involves collecting three-dimensional image data and electrical data from a patient's heart, and then combining these to create a computational model that reflects the heart's time-varying electrical dynamics.

US20210193291A1 shows a computer-based system and process are disclosed for reconstructing the internal electrical behavior of a patient's heart based partly or wholly on the patient's electrocardiogram (ECG).

EP3319521B1 shows a computer-implemented method for creating a map showing how electrical signals travel through the heart.

US20220369930A1 shows systems and methods for identifying cardiac arrhythmia target segments for non-invasive ablation therapy, particularly stereotactic body radiotherapy (SBRT).

US20170330075A1 shows systems and methods for personalizing a cardiac electrophysiology (EP) model using deep learning.

US20210244341A1 shows systems and methods for localizing arrhythmias, particularly ventricular arrhythmias such as premature ventricular contractions (PVCs) or ventricular tachycardia (VT), using ECG data combined with selected 3D heart models.

This application addresses the problem of improving cardiac diagnostic analysis by integrating advanced algorithms into existing clinical workflows without disrupting the workflow process.

The application provides solutions for enhancing the accuracy and efficiency of cardiac diagnostic analysis by employing advanced algorithms to calculate the propagation of electrical excitation across the heart using standard 12-lead ECG data. Panoramic electroanatomical maps of the heart calculated by means of analytical methods.

The present application uses clinical data from surface ECGs, and the results are shown or evaluated in maps. In other words, the present application aims for a patient specific model that closely represents a given surface ECG episode.

The present application is customized for individual patients, by using a mathematical solution that may be tailored for individual patient conditions.

The present application represents electroanatomical maps in both, ventricular and atrial chambers of the heart.

The present application enables panoramic electroanatomical mapping using standard 12-lead ECG as the sole primary input. This approach bypasses the complexity of multimodal imaging, providing a cost-effective, readily available input modality for full 3D cardiac activation analysis.

The present application supports cardiac analysis without requiring changes to existing clinical workflows, ensuring seamless adoption and reducing training overhead.

The present application produces comprehensive electroanatomical activation maps, providing actionable diagnostic insights.

The present application employs a finite element method to model the relationship between body-surface ECG signals and internal cardiac electrical sources, offering a mathematically rigorous approach to inverse problem solving.

The present application utilizes standard 12-lead electrocardiogram (ECG) recordings as the sole input modality for constructing three-dimensional electroanatomical representations. By eliminating the need for specialized electrode vests typically used in electrocardiographic imaging (ECGI), the invention enables streamlined diagnostic procedures that can be performed within existing clinical infrastructure, thereby facilitating broader adoption in routine cardiac care settings.

The present application is designed for integration into existing clinical workflows without necessitating any procedural modifications, ensuring that clinicians can obtain advanced electrophysiological insights with minimal operational disruption, thus enhancing diagnostic throughput and user adoption.

The present application is architected to function with a variety of ECG data sources. This technical flexibility ensures compatibility with standard diagnostic equipment and allows clinicians to apply the system in diverse clinical contexts, regardless of institutional imaging protocols or hardware configurations.

The present application supports direct incorporation into established clinical workflows without requiring changes. This facilitates seamless, diagnostic support and decision-making during routine procedures, increasing clinical adoption and usability.

The present application enables computation of detailed panoramic electroanatomical maps from ECG-based models. These maps provide a comprehensive view of cardiac electrical dynamics and support advanced diagnostics.

The present application utilizes standard 12-lead ECG signals as the central input for deriving 3D maps of cardiac excitation propagation. This approach capitalizes on widely available data, making advanced electroanatomical mapping accessible and practical in diverse clinical settings.

The present application discloses the feature of diagnostics based on standard clinical equipment such as a 12-lead ECG enabling seamless integration into routine clinical workflows. This facilitates the adoption of the system in everyday hospital operations without requiring separate or specialized environments.

The present application discloses the feature of producing interpretable, function-based outputs enabling immediate therapeutic relevance. This supports clinicians through actionable visualizations, reducing reliance on complex interpretation of raw ECG data.

The present application discloses the feature of refining patient-specific electrophysiological models using intra-procedural signal data enabling the system to improve diagnostic precision during an intervention. This supports a personalized treatment approach by dynamically adapting the model based on live electrophysiological feedback.

The present application discloses the feature of solving the inverse problem using a finite element solver to derive cardiac internal activity from observed ECGs enabling a physiologically grounded reconstruction of electrical activity. This represents a clinically aligned modeling approach, as it begins from observable patient data rather than simulated training outputs.

The present application discloses models which are designed for analysis and are seamlessly embedded within existing clinical workflows. This allows clinicians to use the models without altering their standard procedures, enhancing usability and clinical adoption.

The present application computes the relationship between cardiac electrical sources and body-surface ECG signals using lead field approach combined with the finite element method. This enables precise non-invasive localization of electrical activity within the heart, which supports more accurate diagnostics and treatment targeting.

The present application is designed for integration with existing clinical workflows and diagnostic systems. This compatibility allows for a more streamlined implementation and may reduce the need for procedural changes or additional hardware.

The present application utilizes a lead field approach combined with the finite element method framework to derive cardiac source activity from surface ECG data with high mathematical precision. This methodological rigor improves the accuracy of inverse electrophysiological reconstructions.

Embodiments of the invention are associated with various advantages and/or technical effects.

There is disclosed a computer-implemented method for generating a three-dimensional activation map of cardiac tissue based on a 12-lead electrocardiogram (ECG). The method comprises receiving 12-lead ECG data of a patient, selecting at least one cardiac cycle from the ECG data corresponding to an arrhythmic morphology, estimating activation times across cardiac tissue based on the selected cardiac cycle, generating a correlation or any other similarity metric between the selected ECG data and precomputed simulated 12-lead ECG signals stored in a database, wherein each simulated 12-lead ECG signal is associated with a simulated activation map, selecting at least one simulated 12-lead ECG signal based on the correlation metric and retrieving the associated activation map(s), generating an initial activation map by averaging the activation maps of the selected ECG signals, generating a final activation map that best fits the measured 12-lead ECG data by optimizing one or more parameters defining the activation map, the one or more parameters including at least one of regional conduction velocity, fiber orientation, and cardiac anisotropy, and displaying the final activation map using a 3D anatomical representation of the heart.

This application addresses the challenge of improving cardiac diagnostic analysis by integrating advanced algorithms into existing clinical workflows in a non-disruptive manner.

It enhances both accuracy and efficiency by employing advanced algorithms to calculate the propagation of electrical excitation across the heart, based on standard 12-lead ECG data. Panoramic electroanatomical maps of the heart are generated using 12-lead ECG data.

Using clinical data from surface ECGs, the results are visualized and evaluated in map form. The goal is to generate patient-specific models that closely reflect the individual surface ECG episode.

A mathematical solution is employed, which may be customized to account for individual patient conditions. The application enables panoramic electroanatomical mapping based solely on standard 12-lead ECG data, thereby avoiding the complexity and cost associated with multimodal imaging, while still providing a viable input modality for full 3D cardiac activation analysis.

The application supports analysis without necessitating changes to existing clinical workflows, thereby facilitating seamless integration and minimizing training requirements.

There is further disclosed a computer-implemented method for generating a cardiac activation map. The method comprises receiving data for at least one cardiac cycle obtained from ECG data of a patient, determining at least one parameter representing cardiac activation based on the received data, obtaining from a database at least one activation map, wherein the obtained at least one activation map is selected from the database based on the determined at least one parameter, generating an initial activation map based on the at least one activation map obtained from the database, and generating a final activation map by optimizing the initial activation map.

This application aims to improve cardiac diagnostic analysis by embedding advanced algorithms into current clinical workflows, without disrupting established procedures.

It enhances the accuracy and efficiency of cardiac diagnostics through the use of advanced algorithms that compute the propagation of electrical excitation across the heart from standard 12-lead ECG data. Based on these computations, personalized 3D models of the heart's excitation dynamics are generated.

The resulting 3D electroanatomical maps deliver a more physiologically representative visualization of cardiac activity, enabling healthcare professionals to integrate this information seamlessly into their ongoing clinical routines. This ensures smooth implementation and minimizes the need for additional training.

By utilizing standard 12-lead ECG data alone, the application facilitates panoramic electroanatomical mapping, offering a cost-efficient, widely available alternative to more complex multimodal imaging systems.

This approach aims to create patient-specific models that closely replicate the surface ECG episodes observed. It employs a mathematical solution that is adaptable to individual patient conditions, thus supporting a comprehensive, personalized, and integrated strategy in cardiac diagnostics and ultimately contributing to improved patient outcomes.

In a development, the method further comprises receiving ECG data of the patient.

In a development, the ECG data is 12-lead ECG data.

In a development, the method further comprises selecting the at least one cardiac cycle from the ECG data.

In a development, the at least one cardiac cycle is selected corresponding to an arrhythmic morphology.

In a development, the at least one parameter representing cardiac activation is activation times across cardiac tissue.

In a development, obtaining the at least one activation map from the database comprises comparing the selected at least one cardiac cycle to ECG data stored in the database.

In a development, the ECG data stored in the database comprises precomputed simulated ECG signals.

In a development, the step of comparing comprises generating a correlation metric between the selected at least one cardiac cycle and at least one of the precomputed simulated ECG signals.

In a development, the step of comparing further comprises selecting at least one precomputed simulated ECG signal based on the correlation metric and obtain the at least one activation map which is associated with the selected at least one precomputed simulated ECG signal.

In a development, generating the initial activation map comprises averaging the at least one obtained activation maps.

In a development, generating the final activation map comprises optimizing one or more parameters of the initial activation map.

In a development, the one or more parameters include at least one of conduction velocity, fiber orientation, and cardiac anisotropy.

In a development, the method further comprises outputting the activation map to a user.

There is further disclosed a system for generating a cardiac activation map. The system comprises an input interface configured to receive data for at least one cardiac cycle obtained from ECG data of a patient, a database configured for storing at least one activation map, and a processor. The processor is configured to determine at least one parameter representing cardiac activation based on the received data, obtain from the database at least one activation map, wherein the obtained at least one activation map is selected from the database based on the determined at least one parameter, generate an initial activation map based on the at least one activation map obtained from the database, and generate a final activation map by optimizing the initial activation map.

In a development, the system further comprises an output interface for outputting the activation map to a user.

There is further disclosed a computer program comprising instructions which, when the program is executed by a computer, cause the computer to carry out the above method.

There is further disclosed a computer-readable medium comprising instructions which, when executed by a computer, cause the computer to carry out the above method.

There is further disclosed a data processing apparatus, comprising means for carrying out the above method.

There is further disclosed a method for cardiac diagnostic analysis, comprising:
a. receiving standard 12-lead ECG data from a patient;
b. employing advanced algorithms to process the ECG data to calculate the propagation of electrical excitation across the heart;
c. providing analysis of the underlying heart conditions;
d. integrating this analysis into existing clinical workflows without changing those workflows.

The method enhances diagnostic accuracy by utilizing advanced algorithms to calculate the electrical activity of the heart, providing a more detailed understanding of cardiac function.

Integration into existing clinical workflows without the need for changes facilitates seamless adoption of the technology, minimizing disruption to healthcare providers and institutions.

The method provides the advantage of conducting cardiac diagnostic analysis using standard 12-lead ECG data, which is a widely available and commonly used diagnostic tool.

The method provides the advantage of integrating the analysis of the heart models into existing clinical workflows without requiring any changes to those workflows. This ensures seamless integration of the diagnostic analysis into the healthcare professionals' routine practices.

Generating panoramic electroanatomical maps provides a comprehensive view of the heart's electrical activity, aiding in the identification of arrhythmias and other abnormalities.

The panoramic maps enhance the ability to visualize and understand complex cardiac phenomena, which may lead to more effective interventions.

The detailed insights provided by these maps can facilitate the identification of the precise locations of cardiac dysfunctions.

In a development, the method further comprises employing a finite element solver to establish a relationship between cardiac electrical sources and ECGs measured on the body surface.

Employing a finite element solver to establish the relationship between cardiac electrical sources and body surface ECGs allows for non-invasive mapping of cardiac electrophysiological events, which can reduce the need for invasive diagnostic procedures.

The use of a finite element solver enhances the computational efficiency of solving the inverse problem of electrocardiography, enabling faster processing times.

In a development, the method further comprises generating panoramic electroanatomical maps.

In a development, the method further comprises employing a finite element solver in the form of a bilateral-element solver based on a mathematical model that uses finite elements to establish a relationship between cardiac electrical sources and ECGs measured on the body surface. A relationship between cardiac electrical sources and the standard 12-lead ECGs measured on the body surface is then established.

There is further disclosed a computer-implemented method for generating a three-dimensional activation map of cardiac tissue of a patient based on non-invasive 12-lead electrocardiogram (ECG) interpretation without other signals, the method comprising:
receiving 12-lead ECG data of a patient;
selecting at least one cardiac cycle from the ECG data corresponding to an arrhythmic morphology;
estimating activation times across cardiac tissue based on the selected cardiac cycle;
generating a correlation metric between the selected cardiac cycle and a plurality of precomputed simulated 12-lead ECG signals stored in a database, wherein each simulated 12-lead ECG signal is associated with a simulated activation map;
selecting at least one simulated 12-lead ECG signal based on the correlation metric and retrieving the associated activation map(s);
generating an initial activation map based on the at least one retrieved simulated activation map;
generating a final activation map that best fits the measured 12-lead ECG data by optimizing one or more parameters of the initial activation map, the one or more parameters including at least one of conduction velocity, fiber orientation, and cardiac anisotropy; and
displaying the final activation map using a 3D anatomical representation of the heart.

In a development, the method is performed without acquiring patient-specific tomographic imaging data.

In a development, the method is performed without acquiring additional body-surface electrical signals other than the 12-lead ECG data.

In a development, generating the final activation map comprises solving an inverse electrocardiographic problem relating body-surface ECG measurements to cardiac electrical sources.

In a development, solving the inverse electrocardiographic problem comprises using a finite element solver to model a relationship between cardiac electrical sources and ECG signals measured on a body surface.

In a development, the method further comprises selecting a heart model from a plurality of pre-defined anatomical heart models stored in a database.

In a development, the heart model is selected based on at least one patient-specific parameter including gender and estimated heart position within a torso.

In a development, the selected heart model comprises a three-dimensional anatomical representation including a tetrahedral mesh.

In a development, the final activation map is generated using the selected heart model without requiring patient-specific imaging data.

In a development, the precomputed simulated 12-lead ECG signals stored in the database are generated by solving an anisotropic eikonal equation on a three-dimensional heart model to compute activation times and computing corresponding 12-lead ECG signals using a lead field method.

In a development, activation source locations and conduction velocity tensors are varied across multiple simulations.

In a development, the simulations are repeated according to a quasi-random sampling scheme.

In a development, for each simulation the database stores the activation map, the corresponding simulated 12-lead ECG signals, and metadata describing simulation parameters.

In a development, the correlation metric comprises a Spearman correlation coefficient.

In a development, optimizing the electrophysiological parameters comprises adjusting at least one of regional conduction velocity, myocardial fiber orientation, and cardiac anisotropy.

In a further aspect, a system is provided for generating a three-dimensional electroanatomical activation map of a heart of a patient based on non-invasive 12-lead electrocardiogram interpretation without other signals, the system comprising an input interface configured to receive standard 12-lead ECG data of the patient, a database configured to store a plurality of precomputed simulated 12-lead ECG signals each associated with at least one simulated activation map, a processor configured to select at least one cardiac cycle corresponding to an arrhythmic morphology, estimate activation times across cardiac tissue, generate a correlation metric, select at least one simulated 12-lead ECG signal based on the correlation metric and retrieve the associated simulated activation map, generate an initial activation map, and generate a final activation map by optimizing one or more electrophysiological parameters, and an output interface configured to display the final activation map using a three-dimensional anatomical representation of the heart.

In a development, the processing circuitry is configured to select at least two simulated 12-lead ECG signals based on the correlation metric, retrieve at least two associated simulated activation maps, and generate the initial activation map by averaging the at least two retrieved simulated activation maps corresponding to the highest correlation metric values.

In a development, the system is configured to operate without acquiring patient-specific tomographic imaging data.

In a development, the system is configured to operate without acquiring additional body-surface electrical signals other than the 12-lead ECG data.

In a development, the processing circuitry is configured to solve an inverse electrocardiographic problem relating body-surface ECG measurements to cardiac electrical sources.

In a development, the processing circuitry comprises or is configured to use a finite element solver to model a relationship between cardiac electrical sources and ECG signals measured on a body surface.

In a development, the processing circuitry is further configured to select a heart model from a plurality of pre-defined anatomical heart models stored in a database.

In a development, the heart model is selected based on at least one patient-specific parameter including gender and estimated heart position within a torso.

In a development, the selected heart model comprises a three-dimensional anatomical representation including a tetrahedral mesh.

In a development, the simulated 12-lead ECG signals stored in the database are generated by solving an anisotropic eikonal equation on a three-dimensional heart model to compute activation times and computing corresponding 12-lead ECG signals using a lead field method.

In a development, activation source locations and conduction velocity tensors are varied across multiple simulations.

In a development, for each simulation the database stores the activation map, the corresponding simulated 12-lead ECG signals, and metadata describing simulation parameters.

The 3D Modeling Software according to the application comprises several modules.

12-lead ECG data is loaded. Time-series for any number of selected leads can be visualized. There are multiple visualization options including scaling, zooming, scrolling, interval selection. Peaks are found based on the wavelet decomposition of the whole ECG signal. The templates found are then used for reliable peaks' thresholding.

Maps module 25: Given any appropriate 3D heart model and selected ECG fragment, a 3D panoramic electroanatomical map can be created. By selecting different time intervals one can re-compute or compute new maps, save them individually or as the whole project.

Unlike existing methods that require complex electrode setups, this system works with a pre-defined heart model, and standard 12-lead-ECG configurations.

The goal of the invention is a software system that simplifies and enhances cardiac diagnostic processes through non-invasive methods. It uses standard ECG data and advanced algorithms to construct personalized 3D electroanatomical models. It integrates various data types, without altering clinical procedures, and it offers insights for decision-making rather than prescribing specific interventions.

The application works with standard ECG and possibly textbook heart models, streamlining clinical processes without requiring new ones. The 3D modeling software does not aim to replace medical expertise but rather to enhance it, providing visualization and analytical tools to aid decision-making without making clinical decisions itself. This respects the conservative nature of medical adoption and focuses on assisting rather than automating clinical judgments. The software system according to the application makes it easier and better to diagnose heart problems. It takes normal 12-lead-ECG data (a test that checks how your heart is doing) and uses smart algorithms to create personalized 3D models of the excitation spreading in the heart. Unlike other methods that need complicated setups, this system also works with standard - i.e. pre-defined and stored in a database - heart models and can analyze data in real time.

For processing the map creation step, the relation between cardiac electrical sources and ECGs measured on the body surface is provided by the Poisson equation, which is solved with the dedicated finite element solver. The volume conductor is assumed to be homogeneous, however any number of inhomogeneities can be incorporated into the model and available data from functional imaging. As a result, a non-linear relationship is established between activation time on the cardiac surface and the ECG at the respective electrode positions. This non-linear equation is solved with the dedicated optimization routine.

Generating the output comprises the following steps:
a) distinct panoramic maps showing physiological scalar (e.g. activation times) or vector (e.g. propagation) maps
b) local arrhythmogenic sources
c) ECG characteristics (e.g. duration of waves, fractionation indices)

The application delivers a holistic view on the cardiac state of condition and can be used for improved guidance and therefore success rates of therapies, both acutely and long-term.

The application takes into account that
1) the electrical signals coming from the heart to the body surface are affected by patient-specific heart anatomy and position, and that
2) intracardiac interventions also do require the anatomy of cardiac chambers to map out the most probable excitation pathways.

According to the application, ECG is used for providing a global panoramic picture of electrical excitation in the heart, restricting analysis to the local area of catheter manipulations. Mapping of the whole chamber is performed in a consecutive way by mapping out local zones of potential interest.

The device of the application provides non-invasive 12-lead ECG interpretation without other signals.

The 3D modeling software according to the application comprises the following software modules:
Automated analysis of ECG 21
Automated reconstruction of 3D maps based on standard tools and processes
All modules run locally on standard computer hardware.

The 3D modeling software fills the crucial niches hindering adoption of patient-centric cost-effective treatment strategies.

The provided upgrade is ensured by
1) the approach based on standard processes and tools, thus not requiring additional logistical efforts critical in the clinical environment
2) automated evaluation of all components with the insights for all involved healthcare units - resident general practitioners, cardiologists, hospitals, telemedicine centers, insurance companies
3) time and cost reduction together with lower readmission numbers, allowing better services to the whole population
4) generally, a more holistic approach to cardiovascular health via digital advances.

The software of the application allows the better use of existing hardware for cardiac arrhythmia diagnosis - 3-dimensional electro-anatomical maps of the heart - by interfacing with existing hardware and automatically integrating with standard non-invasive processes, significantly improving patient outcomes and healthcare provider efficiency.

The application creates 3-dimensional electro-anatomical maps of the heart based on conventional 12-lead ECG recordings, by simply interfacing to existing ECG-hardware. These maps can be used in the diagnosis of cardiac arrhythmias such as atrial fibrillation. Until now, these maps could only be created invasively during a catheter procedure or using complex electrode vests with around 200 electrodes.

The results are made available to everyone involved in the healing. This significantly improves the patient's prognosis and makes the healing process more efficient. This and the simplified data exchange and generation of patient-specific data result in significant cost savings.

An example implementation of a method according to the invention is described below. The method can comprise the steps patient data acquisition and upload into the system, map generation, and visualization and analysis.

Patient data is acquired and uploaded into the system as described in the following. A digital 12-lead ECG is obtained during routine clinical evaluation or prior to an electrophysiological intervention. The ECG is recorded in accordance with applicable clinical standards and exported in a digital format compatible with the system (e.g., XML, DICOM).

The ECG file is uploaded to a mapping system interface wherein a user (e.g., a physician or technician) selects one or more cardiac cycles exhibiting the arrhythmic morphology of interest. Selection may be performed manually or semi-automatically via built-in tools that facilitate beat identification). Table 1 below shows an example of the numerical data of 12 standard-channels for such an interval at 1kHz sampling rate (values are provided in microvolts).

**Table 1: example of numerical data of 12 standard-channels at 1kHz sampling rate**

| **I** | **II** | **III** | **AvR** | **AvL** | **AvF** | **V1** | **V2** | **V3** | **V4** | **V5** | **V6** |
|---|---|---|---|---|---|---|---|---|---|---|---|
| -29 | -69 | -37 | 50 | 1 | -52 | 23 | -32 | -51 | -69 | -71 | -72 |
| -27 | -67 | -37 | 48 | 4 | -53 | 21 | -33 | -52 | -68 | -68 | -69 |
| -28 | -68 | -38 | 49 | 4 | -55 | 17 | -36 | -56 | -69 | -68 | -68 |
| -29 | -72 | -39 | 52 | 3 | -56 | 13 | -41 | -60 | -71 | -68 | -66 |
| -32 | -74 | -40 | 54 | 3 | -57 | 9 | -45 | -65 | -73 | -67 | -65 |
| -31 | -75 | -40 | 56 | 3 | -59 | 4 | -50 | -70 | -74 | -67 | -63 |
| -29 | -78 | -42 | 57 | 4 | -62 | 0 | -55 | -75 | -77 | -67 | -62 |
| -29 | -80 | -45 | 58 | 6 | -62 | -7 | -60 | -81 | -80 | -68 | -61 |
| -32 | -80 | -46 | 57 | 7 | -62 | -12 | -65 | -85 | -81 | -67 | -59 |
| -33 | -78 | -48 | 55 | 9 | -63 | -17 | -69 | -89 | -83 | -66 | -56 |
| -35 | -76 | -51 | 51 | 13 | -64 | -22 | -74 | -94 | -84 | -64 | -52 |
| -35 | -76 | -54 | 49 | 16 | -66 | -29 | -78 | -98 | -86 | -62 | -49 |
| -34 | -77 | -57 | 48 | 18 | -66 | -36 | -83 | -103 | -88 | -61 | -46 |
| -33 | -74 | -59 | 44 | 22 | -65 | -42 | -86 | -107 | -89 | -58 | -43 |
| -30 | -67 | -63 | 35 | 29 | -62 | -47 | -88 | -108 | -88 | -53 | -37 |
| -25 | -58 | -66 | 23 | 38 | -60 | -53 | -91 | -110 | -87 | -48 | -31 |
| -21 | -50 | -70 | 13 | 45 | -57 | -60 | -95 | -112 | -86 | -43 | -25 |
| -19 | -41 | -71 | 4 | 51 | -50 | -65 | -97 | -112 | -83 | -35 | -17 |
| -14 | -28 | -69 | -7 | 55 | -42 | -71 | -97 | -111 | -78 | -25 | -7 |
| -2 | -15 | -67 | -19 | 59 | -34 | -75 | -97 | -110 | -72 | -14 | 3 |
| 10 | 0 | -64 | -32 | 64 | -25 | -79 | -97 | -107 | -64 | -2 | 14 |
| 22 | 14 | -62 | -46 | 68 | -18 | -84 | -97 | -105 | -56 | 9 | 26 |
| 32 | 25 | -59 | -55 | 70 | -12 | -92 | -99 | -104 | -49 | 21 | 37 |
| 43 | 33 | -55 | -60 | 69 | -4 | -101 | -101 | -103 | -41 | 33 | 48 |
| 54 | 43 | -50 | -67 | 69 | 5 | -108 | -102 | -100 | -31 | 47 | 61 |
| 66 | 56 | -44 | -77 | 69 | 12 | -113 | -101 | -95 | -18 | 61 | 75 |
| 78 | 66 | -42 | -84 | 71 | 17 | -121 | -100 | -91 | -7 | 74 | 86 |
| 84 | 74 | -41 | -92 | 74 | 23 | -128 | -99 | -86 | 3 | 85 | 95 |
| 87 | 83 | -39 | -100 | 76 | 30 | -136 | -96 | -79 | 16 | 98 | 106 |
| 91 | 94 | -35 | -109 | 78 | 39 | -141 | -92 | -70 | 30 | 112 | 118 |
| 98 | 107 | -31 | -120 | 82 | 48 | -145 | -85 | -60 | 45 | 126 | 130 |
| 103 | 122 | -27 | -134 | 86 | 59 | -149 | -79 | -49 | 62 | 141 | 143 |
| 110 | 138 | -22 | -148 | 90 | 68 | -153 | -73 | -37 | 79 | 158 | 157 |
| 117 | 151 | -16 | -158 | 90 | 77 | -157 | -66 | -25 | 96 | 174 | 170 |
| 124 | 163 | -10 | -168 | 91 | 87 | -161 | -58 | -12 | 112 | 189 | 183 |
| 134 | 177 | -2 | -178 | 90 | 97 | -162 | -49 | 1 | 129 | 204 | 197 |
| 146 | 189 | 6 | -186 | 88 | 109 | -162 | -38 | 16 | 145 | 219 | 210 |
| 158 | 201 | 16 | -193 | 84 | 121 | -159 | -25 | 34 | 163 | 234 | 222 |
| 166 | 214 | 28 | -201 | 79 | 132 | -155 | -9 | 55 | 183 | 250 | 236 |
| 173 | 228 | 38 | -209 | 76 | 141 | -151 | 7 | 77 | 203 | 266 | 249 |
| 180 | 240 | 43 | -218 | 77 | 149 | -149 | 23 | 98 | 222 | 281 | 260 |
| 184 | 253 | 46 | -229 | 80 | 157 | -149 | 41 | 120 | 243 | 295 | 271 |
| 186 | 267 | 47 | -241 | 84 | 167 | -147 | 60 | 144 | 265 | 311 | 283 |
| 188 | 283 | 51 | -256 | 88 | 181 | -146 | 80 | 169 | 291 | 330 | 298 |
| 191 | 303 | 60 | -271 | 88 | 197 | -144 | 102 | 198 | 322 | 355 | 318 |
| 198 | 325 | 69 | -287 | 88 | 212 | -144 | 125 | 228 | 357 | 383 | 340 |
| 207 | 346 | 78 | -304 | 90 | 228 | -147 | 147 | 259 | 396 | 414 | 364 |
| 218 | 368 | 86 | -322 | 93 | 245 | -151 | 168 | 290 | 436 | 447 | 389 |
| 229 | 393 | 97 | -341 | 94 | 269 | -153 | 193 | 326 | 482 | 484 | 418 |
| 239 | 420 | 116 | -359 | 89 | 292 | -151 | 225 | 368 | 534 | 527 | 450 |
| 251 | 446 | 136 | -376 | 83 | 313 | -148 | 258 | 413 | 589 | 570 | 483 |
| 263 | 471 | 155 | -391 | 79 | 335 | -147 | 291 | 460 | 645 | 614 | 515 |
| 277 | 497 | 173 | -408 | 76 | 358 | -144 | 328 | 512 | 706 | 659 | 547 |
| 291 | 525 | 190 | -429 | 75 | 383 | -139 | 367 | 568 | 769 | 706 | 579 |
| 299 | 555 | 210 | -449 | 71 | 408 | -132 | 411 | 628 | 834 | 754 | 613 |
| 306 | 582 | 233 | -465 | 63 | 431 | -123 | 457 | 690 | 901 | 801 | 646 |
| 313 | 606 | 256 | -477 | 53 | 455 | -115 | 502 | 754 | 967 | 848 | 678 |
| 322 | 630 | 280 | -490 | 43 | 480 | -106 | 551 | 822 | 1036 | 896 | 711 |
| 334 | 659 | 301 | -508 | 36 | 508 | -96 | 601 | 892 | 1111 | 949 | 747 |
| 344 | 691 | 325 | -528 | 28 | 539 | -87 | 655 | 965 | 1191 | 1008 | 790 |
| 349 | 727 | 350 | -551 | 20 | 567 | -78 | 709 | 1040 | 1275 | 1072 | 836 |
| 350 | 762 | 373 | -574 | 13 | 591 | -73 | 760 | 1112 | 1358 | 1137 | 885 |
| 351 | 791 | 393 | -593 | 7 | 616 | -73 | 804 | 1176 | 1438 | 1201 | 933 |
| 358 | 823 | 410 | -616 | 3 | 644 | -77 | 843 | 1236 | 1517 | 1268 | 984 |
| 367 | 862 | 427 | -647 | 4 | 675 | -82 | 880 | 1294 | 1599 | 1340 | 1041 |
| 377 | 905 | 445 | -680 | 5 | 707 | -88 | 913 | 1347 | 1682 | 1415 | 1100 |
| 389 | 948 | 465 | -712 | 5 | 738 | -99 | 937 | 1393 | 1760 | 1490 | 1161 |
| 397 | 989 | 487 | -743 | 2 | 768 | -111 | 956 | 1434 | 1836 | 1564 | 1222 |
| 410 | 1024 | 512 | -766 | -5 | 800 | -125 | 969 | 1469 | 1907 | 1636 | 1282 |
| 432 | 1060 | 538 | -788 | -14 | 836 | -140 | 975 | 1498 | 1974 | 1708 | 1342 |
| 456 | 1099 | 571 | -811 | -27 | 876 | -157 | 975 | 1518 | 2038 | 1780 | 1404 |
| 478 | 1141 | 610 | -834 | -43 | 918 | -176 | 967 | 1529 | 2097 | 1853 | 1468 |
| 497 | 1183 | 652 | -856 | -62 | 962 | -198 | 949 | 1528 | 2149 | 1925 | 1534 |
| 507 | 1228 | 695 | -879 | -81 | 1005 | -225 | 920 | 1513 | 2191 | 1994 | 1600 |
| 516 | 1276 | 734 | -908 | -93 | 1049 | -258 | 880 | 1484 | 2226 | 2063 | 1667 |
| 523 | 1323 | 773 | -935 | -107 | 1091 | -298 | 826 | 1439 | 2253 | 2132 | 1735 |
| 527 | 1366 | 816 | -957 | -126 | 1137 | -345 | 758 | 1377 | 2269 | 2197 | 1803 |
| 529 | 1412 | 861 | -981 | -146 | 1184 | -395 | 679 | 1301 | 2276 | 2261 | 1872 |
| 532 | 1460 | 909 | -1005 | -169 | 1232 | -448 | 589 | 1212 | 2275 | 2325 | 1943 |
| 542 | 1506 | 957 | -1027 | -194 | 1278 | -503 | 491 | 1113 | 2266 | 2385 | 2013 |
| 550 | 1553 | 1002 | -1051 | -216 | 1322 | -559 | 389 | 1008 | 2253 | 2441 | 2080 |
| 551 | 1600 | 1044 | -1077 | -236 | 1366 | -614 | 286 | 902 | 2237 | 2496 | 2146 |
| 553 | 1642 | 1091 | -1095 | -264 | 1413 | -666 | 181 | 791 | 2215 | 2545 | 2207 |
| 553 | 1678 | 1148 | -1103 | -305 | 1462 | -713 | 72 | 675 | 2181 | 2584 | 2263 |
| 551 | 1715 | 1210 | -1108 | -352 | 1512 | -755 | -34 | 558 | 2137 | 2614 | 2314 |
| 553 | 1752 | 1271 | -1115 | -396 | 1563 | -794 | -143 | 437 | 2083 | 2637 | 2361 |
| 558 | 1791 | 1334 | -1122 | -441 | 1610 | -831 | -253 | 311 | 2018 | 2652 | 2406 |
| 552 | 1824 | 1396 | -1124 | -489 | 1656 | -866 | -366 | 179 | 1938 | 2656 | 2445 |
| 531 | 1854 | 1457 | -1124 | -536 | 1703 | -900 | -479 | 44 | 1847 | 2653 | 2479 |
| 505 | 1886 | 1520 | -1124 | -583 | 1751 | -931 | -593 | -92 | 1746 | 2642 | 2510 |
| 482 | 1915 | 1587 | -1121 | -635 | 1799 | -961 | -705 | -229 | 1637 | 2622 | 2536 |
| 459 | 1941 | 1656 | -1113 | -689 | 1841 | -989 | -816 | -366 | 1515 | 2589 | 2552 |
| 429 | 1962 | 1720 | -1102 | -741 | 1878 | -1015 | -921 | -501 | 1382 | 2537 | 2555 |
| 399 | 1976 | 1780 | -1087 | -792 | 1911 | -1038 | -1024 | -634 | 1235 | 2462 | 2540 |
| 368 | 1985 | 1836 | -1070 | -840 | 1935 | -1058 | -1119 | -761 | 1079 | 2364 | 2506 |
| 331 | 1987 | 1884 | -1049 | -885 | 1957 | -1074 | -1204 | -878 | 920 | 2246 | 2454 |
| 289 | 1986 | 1929 | -1026 | -929 | 1977 | -1085 | -1279 | -983 | 756 | 2109 | 2386 |
| 246 | 1984 | 1970 | -1004 | -970 | 1990 | -1089 | -1342 | -1076 | 593 | 1961 | 2308 |
| 200 | 1979 | 2002 | -984 | -1004 | 1994 | -1088 | -1395 | -1157 | 429 | 1805 | 2221 |
| 153 | 1966 | 2024 | -960 | -1032 | 1990 | -1085 | -1441 | -1232 | 263 | 1639 | 2124 |
| 107 | 1944 | 2038 | -931 | -1058 | 1981 | -1081 | -1481 | -1300 | 103 | 1472 | 2022 |
| 60 | 1921 | 2044 | -903 | -1078 | 1970 | -1076 | -1513 | -1355 | -43 | 1315 | 1923 |
| 16 | 1898 | 2045 | -879 | -1092 | 1959 | -1068 | -1535 | -1397 | -173 | 1172 | 1831 |
| -20 | 1877 | 2044 | -858 | -1104 | 1951 | -1057 | -1549 | -1429 | -288 | 1045 | 1748 |
| -57 | 1861 | 2042 | -841 | -1113 | 1946 | -1042 | -1554 | -1451 | -386 | 934 | 1674 |
| -94 | 1853 | 2041 | -831 | -1119 | 1940 | -1025 | -1553 | -1463 | -462 | 842 | 1609 |
| -125 | 1843 | 2039 | -820 | -1124 | 1925 | -1008 | -1548 | -1467 | -522 | 762 | 1547 |
| -149 | 1819 | 2032 | -798 | -1130 | 1903 | -992 | -1539 | -1465 | -569 | 688 | 1484 |
| -170 | 1792 | 2016 | -778 | -1128 | 1878 | -974 | -1523 | -1454 | -601 | 624 | 1420 |
| -185 | 1767 | 1989 | -765 | -1114 | 1846 | -950 | -1496 | -1432 | -618 | 568 | 1359 |
| -193 | 1737 | 1955 | -751 | -1094 | 1800 | -920 | -1460 | -1398 | -622 | 522 | 1300 |
| -201 | 1692 | 1909 | -730 | -1069 | 1738 | -886 | -1413 | -1355 | -614 | 482 | 1238 |
| -217 | 1631 | 1845 | -703 | -1033 | 1660 | -847 | -1357 | -1301 | -599 | 447 | 1173 |
| -227 | 1556 | 1763 | -670 | -987 | 1564 | -805 | -1293 | -1241 | -579 | 413 | 1105 |
| -225 | 1463 | 1665 | -629 | -933 | 1455 | -761 | -1224 | -1177 | -559 | 379 | 1033 |
| -220 | 1359 | 1551 | -584 | -869 | 1343 | -715 | -1148 | -1109 | -539 | 344 | 958 |
| -217 | 1255 | 1431 | -542 | -799 | 1227 | -663 | -1067 | -1035 | -515 | 312 | 884 |
| -212 | 1145 | 1309 | -496 | -730 | 1105 | -608 | -980 | -957 | -488 | 284 | 812 |
| -204 | 1030 | 1180 | -447 | -659 | 980 | -552 | -890 | -876 | -460 | 256 | 740 |
| -198 | 912 | 1048 | -397 | -586 | 861 | -496 | -800 | -796 | -432 | 228 | 666 |
| -188 | 804 | 919 | -353 | -513 | 750 | -439 | -713 | -718 | -404 | 201 | 594 |
| -171 | 701 | 800 | -308 | -447 | 643 | -386 | -633 | -645 | -377 | 175 | 524 |
| -159 | 596 | 690 | -258 | -391 | 539 | -338 | -560 | -579 | -355 | 146 | 453 |
| -146 | 496 | 584 | -209 | -337 | 445 | -290 | -490 | -516 | -332 | 118 | 384 |
| -133 | 406 | 486 | -166 | -286 | 363 | -241 | -423 | -455 | -309 | 92 | 319 |
| -115 | 332 | 395 | -135 | -234 | 289 | -193 | -359 | -396 | -283 | 70 | 261 |
| -102 | 266 | 314 | -107 | -187 | 224 | -149 | -304 | -345 | -262 | 48 | 204 |
| -98 | 201 | 247 | -73 | -154 | 169 | -112 | -260 | -305 | -247 | 25 | 150 |
| -93 | 145 | 193 | -42 | -128 | 121 | -81 | -223 | -271 | -234 | 3 | 101 |
| -87 | 96 | 147 | -15 | -106 | 83 | -56 | -193 | -243 | -224 | -17 | 58 |
| -71 | 57 | 110 | 5 | -87 | 53 | -34 | -168 | -221 | -215 | -35 | 21 |
| -56 | 28 | 78 | 18 | -68 | 27 | -16 | -147 | -201 | -207 | -49 | -8 |
| -54 | 0 | 55 | 33 | -56 | 5 | -4 | -134 | -189 | -204 | -63 | -35 |
| -55 | -24 | 35 | 46 | -47 | -11 | 0 | -126 | -181 | -204 | -77 | -58 |
| -57 | -43 | 20 | 55 | -39 | -20 | 2 | -120 | -178 | -206 | -90 | -78 |
| -55 | -51 | 10 | 57 | -31 | -26 | 5 | -114 | -171 | -206 | -98 | -90 |
| -51 | -58 | 5 | 58 | -27 | -32 | 8 | -107 | -165 | -204 | -102 | -97 |
| -52 | -65 | -1 | 60 | -24 | -43 | 9 | -101 | -160 | -203 | -106 | -102 |
| -55 | -75 | -11 | 63 | -19 | -55 | 7 | -98 | -157 | -206 | -112 | -109 |
| -57 | -88 | -21 | 70 | -16 | -63 | 5 | -95 | -155 | -208 | -119 | -117 |
| -55 | -98 | -28 | 76 | -15 | -66 | 7 | -90 | -150 | -209 | -123 | -123 |
| -55 | -99 | -33 | 76 | -13 | -69 | 11 | -83 | -143 | -207 | -125 | -125 |
| -56 | -101 | -36 | 76 | -12 | -77 | 17 | -75 | -136 | -204 | -127 | -127 |
| -58 | -114 | -39 | 90 | -18 | -88 | 21 | -71 | -132 | -205 | -133 | -134 |
| -62 | -130 | -45 | 105 | -22 | -100 | 23 | -70 | -131 | -209 | -142 | -143 |
| -67 | -143 | -56 | 115 | -19 | -108 | 25 | -69 | -131 | -211 | -151 | -153 |
| -66 | -148 | -66 | 117 | -13 | -111 | 31 | | -128 | -212 | -158 | -161 |

When an ECG fragment becomes available, the map generation can be done using a default, or pre-defined average, anatomical model of the heart.

A map is generated as described below, after patient data was acquired and uploaded into the system.

For the creation of 3D electro-anatomical maps from conventional recorded 12-lead-ECGs a database of calculated cardiac activation models and simulated 12-lead-ECGs is created. Creating the database comprises the steps of modeling a cardiac activation model and of simulating numerical 12-lead ECG.

The cardiac electrical activation is modeled using the anisotropic eikonal equation, which simulates the propagation of electrical waves through the myocardium by accounting for anisotropic conduction velocities inherent in cardiac tissue. This equation has activation times (AT) as unknowns and describes how the activation propagates through the tissue, with the conduction velocity tensor capturing the anisotropic nature of myocardial fibers leading to faster excitation spread along the fiber direction compared to across it.

To solve this equation on a tetrahedral domain that represents the complex cardiac geometry, a fast iterative method is implemented as described in Fu Z, Kirby RM, Whitaker RT. A FAST ITERATIVE METHOD FOR SOLVING THE EIKONAL EQUATION ON TETRAHEDRAL DOMAINS. SIAM J Sci Comput. 2013;35(5):c473-c494. doi: 10.1137/120881956. PMID: 25221418; PMCID: PMC4162315. In this approach, the myocardium is discretized into a tetrahedral mesh, and the AT at the mesh nodes are iteratively updated until the convergence is achieved.

Numerical 12-lead ECG is further simulated. Cardiac transmembrane voltages (TMV) are calculated from the AT using a cellular action potential template. The TMV gradients calculated within all tetrahedra in the cardiac mesh are then equivalent to the current dipole source distribution.

For 12-lead ECG simulation, the lead field approach is utilized as described in Potse M. Scalable and Accurate ECG Simulation for Reaction-Diffusion Models of the Human Heart. Front Physiol. 2018 Apr 20;9:370. doi: 10.3389/fphys.2018.00370. PMID: 29731720; PMCID: PMC5920200. In this method, the electrical activity of the heart is modeled as a distribution of current dipoles that generate a potential field throughout the torso. The potentials measured by each lead are obtained by integrating the contributions of the cardiac electrical sources weighted by the corresponding lead field. The lead field for each electrode is derived by solving a corresponding Laplace equation within the torso domain, taking into account the geometry and tissue conductivities. The solution to these equation provide spatial maps describing how potentials generated within the myocardium are projected to the electrode locations on the body surface.

Integration of different source distributions result in 12-lead ECG signals varying in both amplitude and morphology. Thus, by incorporating accurate lead fields computation and cardiac activation models, this simulation approach produces 12-lead ECG signals that reflect spatio-temporal heterogeneity of the cardiac electrical activity.

A simulated dataset containing realistic cardiac electrical activation patterns on a three-dimensional heart/torso domain are further generated. This dataset is constructed by varying key parameters related to the activation source and conduction velocity tensors across the myocardium. Main goal of this step is to simulate a range of physiological and pathophysiological conditions by varying the location and strength of activation sources and by modulating conduction velocities.

For the activation source, e.g., focal excitation is considered, whereby a single point within the myocardium is chosen to serve as the activation trigger. This method allows for simulation of focal activation patterns, effectively mimicking the electrical behavior observed under arrhythmic events.

Modulation of conduction velocity is another aspect of the dataset creation. The model incorporates anisotropic conduction by varying the conduction velocities in the directions along and transversal to the myocardial fiber orientation. This allows the simulations to capture the physiological effect of faster conduction along the fiber and slower conduction across it. To further enrich the simulated dataset, pathological conditions were simulated by introducing regions with reduced conduction velocity that mimic scar tissue or fibrotic areas.

The simulation process involves solution of the forward problem, where the cardiac electrical activation model, based on the anisotropic eikonal equation, was used to compute activation times across the tetrahedral mesh representing the heart. Multiple simulations are run while systematically varying both the activation source and conduction velocity parameters using Halton scheme of quasi-Monte Carlo method. The resulting activation maps, along with the detailed parameter settings such as source locations and conduction velocities, are stored as part of the dataset. Moreover, synthetic (simulated) 12-lead ECG signals were generated using the lead field approach.

The simulation outputs are (as elements of the database) activation Maps, parameter metadata, and simulated ECG signals. The activation maps provide high-resolution datasets containing AT across the myocardium under varied conditions. The parameter metadata provide comprehensive log of the parameter variations for each dataset instance, including details on activation source locations, onset times, and conduction velocity values. The simulated ECG signals provide corresponding 12-lead ECG signals generated via the lead field method.

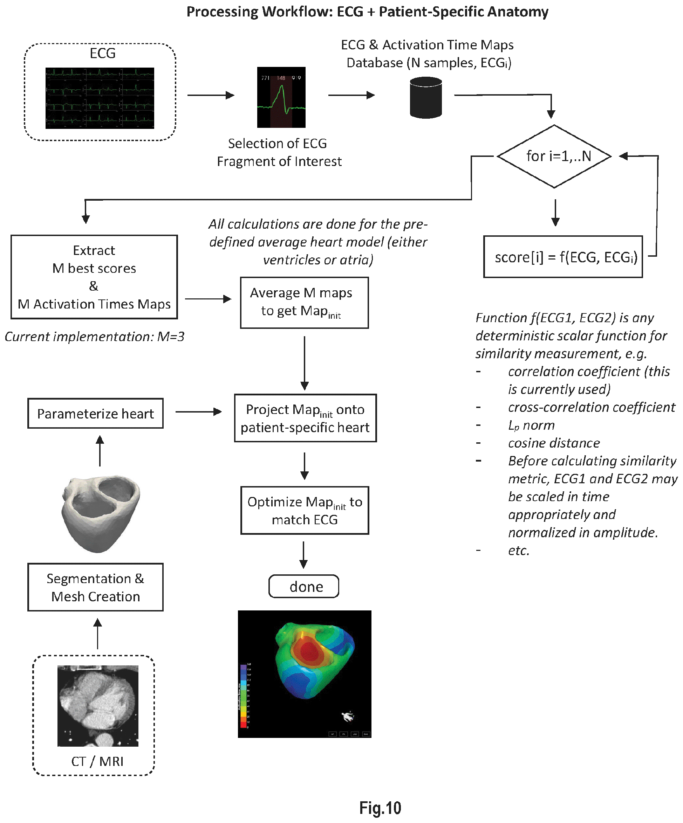

An algorithm for the map generation is described below and is further described in Fig. 10 and Fig. 11 of the present application.

Upon selection of the target interval the system initiates computational solution:
An inverse mapping algorithm is used to identify the best-matching activation maps from the set of simulated ECG signals. The process leverages signal correlation to compare simulated ECG data with the clinical patient's ECG recording. Specifically, the algorithm calculates the correlation between the patient's 12-lead ECG and each simulated 12-lead ECG in the dataset. The top three simulated signals exhibiting the highest correlation are selected, and their corresponding activation maps are retrieved as candidate solutions.

First, the simulated dataset, including activation maps and their associated ECG signals generated via the lead field approach, is organized for efficient comparison. Second, the real patient ECG is pre-processed in the same way as the simulated data to ensure consistency in signal characteristics.

Prior to performing the correlation analysis, a resampling step is applied to the simulated ECG signals to accurately match the QRS complex length. This resampling adjusts the temporal resolution of the simulated ECGs using fast Fourier transform resampling to ensure that the duration and morphology of the QRS complex are properly represented. This normalization step prevents variations in sampling frequency or resolution from distorting the temporal features of the QRS complex, which provide for accurate correlation with the patient's ECG.

Next, a correlation analysis is performed. For each simulated ECG signal, the Spearman correlation coefficient is computed with the real patient's ECG. By ranking the simulated signals based on their correlation scores, the algorithm identifies the top three signals that most closely resemble the patient's ECG.

In the final step, the activation maps corresponding to these top three signals are selected and averaged to produce an activation map ('initial map' in the following), to be used by its own and be subject for further optimization. This averaging helps to reduce noise and enhance the robustness of the estimated activation pattern. The final solution step is the optimization of the activation related parameters to match the calculated and measured ECG.

In the case of using the average heart model, the initial map is taken as an initialization for the optimization routine.

Visualization and analysis can be performed after map generation. The resulting activation map is displayed via the system's visualization module which renders isochronal map and propagational patterns superimposed on the anatomical heart model. The user may interactively examine regions of interest.

### BRIEF DESCRIPTION OF DRAWINGS

The present disclosure is illustrated by way of example and not limited in the accompanying figures in which like reference numerals indicate similar elements. Embodiments of the application will now be described with reference to the attached drawings:
Figure 1 shows a patient that is connected to a system of the application;
Figure 2 shows a schematic overview of the 3D modeling software of Fig. 1.
Figure 3 shows a flowchart of a method for time course comparison of 3D electroanatomic maps for arrhythmia monitoring.

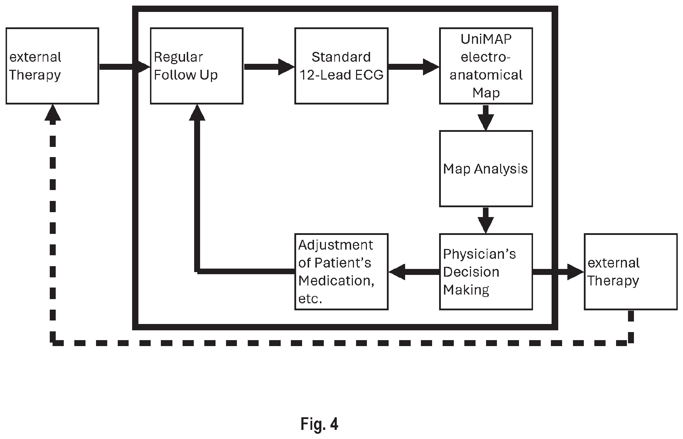

Figure 4 shows a treatment sequence of a patient.
Figure 5 shows a flowchart of a method for generating a cardiac activation map.
Figure 6 shows a flowchart of a method for non-invasive cardiac mapping.
Figure 7 shows a flowchart of a method for analyzing ECG signals for use in non-invasive cardiac mapping.
Figure 8 shows a flowchart of a method for generating a three-dimensional activation map of cardiac tissue based on a 12-lead electrocardiogram (ECG).
Figure 9 shows a flowchart of a further method for generating a three-dimensional activation map of cardiac tissue based on a 12-lead electrocardiogram (ECG).
Figure 10 shows a flowchart of a further method for generating a three-dimensional activation map of cardiac tissue based on a 12-lead electrocardiogram (ECG).

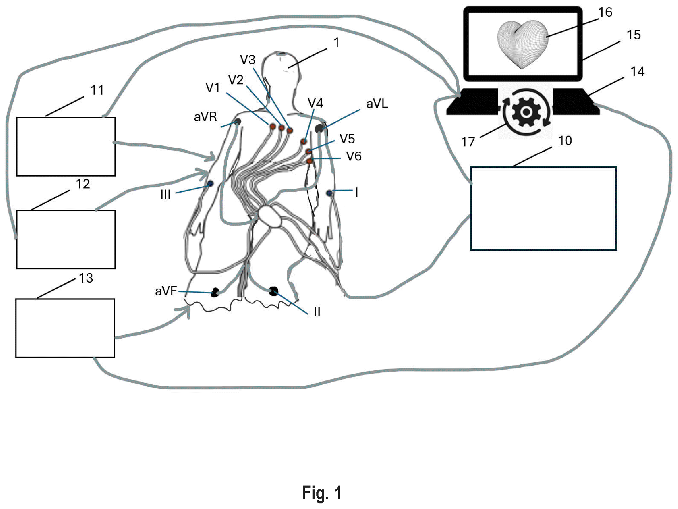

Figure 1 illustrates a comprehensive system designed for cardiac diagnostics. The patient 1 is centrally depicted with various connections to diagnostic equipment. These include an ECG machine 10 directly connected to the patient, displaying ECG data through various leads labeled I, II, III, aVR, aVL, aVF, V1 through V6.

The figure demonstrates the components and workflow of a method for cardiac diagnostic analysis. At the center, there is a representation of a patient 1, from whom standard 12-lead ECG data 30 is being collected. Leads labeled with V1 to V6, aVR, aVL, and aVF along with I, II, III indicate the various positions on the patient's body where ECG data is captured. On the right side of the figure, we see a data integration hub computer 14 with a computer screen 15 displaying a 3D model of the heart 16. This implies that the ECG data and imaging data are processed and integrated to construct this heart model. The ECG machine 10 is symbolically represented, suggesting the software's capability to process the ECG data. The combination of these elements showcases a comprehensive system designed to provide analysis of cardiac health, supporting professionals in the diagnosis and treatment of heart-related conditions.

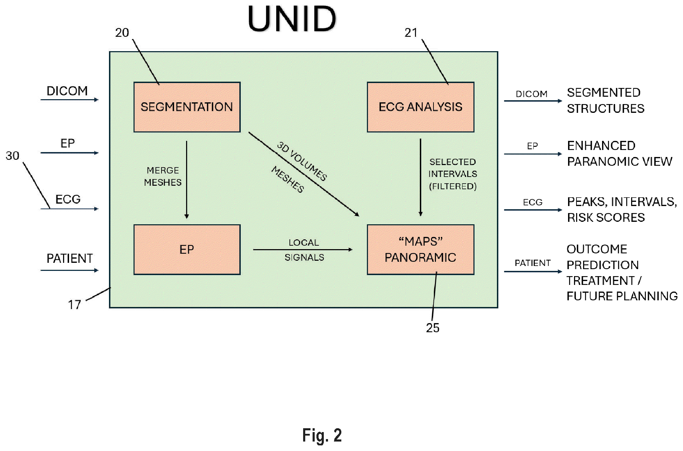

Figure 2 depicts the workflow of the 3D modeling software "CorLector" 17, focusing on the integration and analysis of diverse medical data for cardiac care. The diagram features several modules and data pathways, and it shows a visualization of "CorLector"'s data flow, from input to analysis and output. It covers the different types of data inputs (ECG, patient history), how they're processed (segmentation, merging, analysis), and the types of outputs generated (EP maps, ECG analysis).

The core of the system is represented by the primary ECG analysis module 21. The segmentation module 20 processes the anatomical model of the heart. This module receives inputs from electrophysiological (EP) data, and ECG data 30, and is closely linked to the patient 1.

The ECG analysis module 21 focuses on processing ECG data to identify critical intervals and peaks which are crucial for accurate cardiac analysis. The outputs from this module feed into the "MAPS" module 25, which is tasked with generating panoramic views of the heart's electrical activity.

The maps module 25 integrates outputs from ECG analysis modules to produce comprehensive electroanatomical maps. These maps provide detailed visualizations of segmented structures, enhanced panoramic views, and specific cardiac metrics such as peaks, intervals, and risk scores.

The block diagram is labeled "CorLector" at the top, which indicates a system or component name. Inputs on the left side include ECG data 30, patient information 1. These inputs feed into two primary modules: the segmentation module 20 and the ECG analysis module 21. Subsequently, these 3D volumes merge with electrophysiological data (EP) to produce electrophysiological maps (EP) indicating local signals. This process aligns with claim 4, which involves generating panoramic electroanatomical maps using personalized 3D models, On the right side, the ECG analysis module 21 processes ECG data to filter and select specific intervals, which then feed into a "MAPS" panoramic module 25. It is not explicitly labeled in the figure but likely corresponds to the maps module mentioned in the claim set. Outputs from the system are categorized by data type on the right side. Electro-physiological (EP) inputs lead to an "ENHANCED PANORAMIC VIEW" which could be related to the panoramic electroanatomical maps (claim 5). The ECG analysis provides "PEAKS, INTERVALS, RISK SCORES," capturing elements of the ECG that could be deemed significant for diagnostics and potential risk assessment. This could correlate with claim 10, which mentions employing a finite element solver. Lastly, patient inputs contribute to "OUTCOME PREDICTION TREATMENT / FUTURE PLANNING," suggesting that the system's analysis offers prognostic insight and aids in determining the course of treatment or management for the individual.

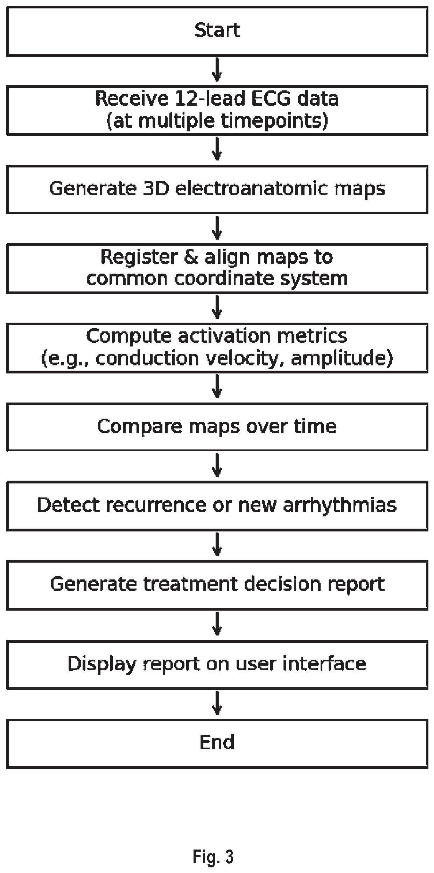

Figure 3 shows a flowchart of a method for time course comparison of 3D electroanatomic maps for arrhythmia monitoring. The method comprises the step of receiving 12-lead ECG data 30 at multiple timepoints such as a set 32 of ECG data comprising a first set 34 of ECG data at a first timepoint and a second set 36 of ECG data at a second timepoint. The method further comprises generating 3D electroanatomic maps such as a first electroanatomic map 40 representing the first timepoint and a second electroanatomic map 42 representing the second timepoint.

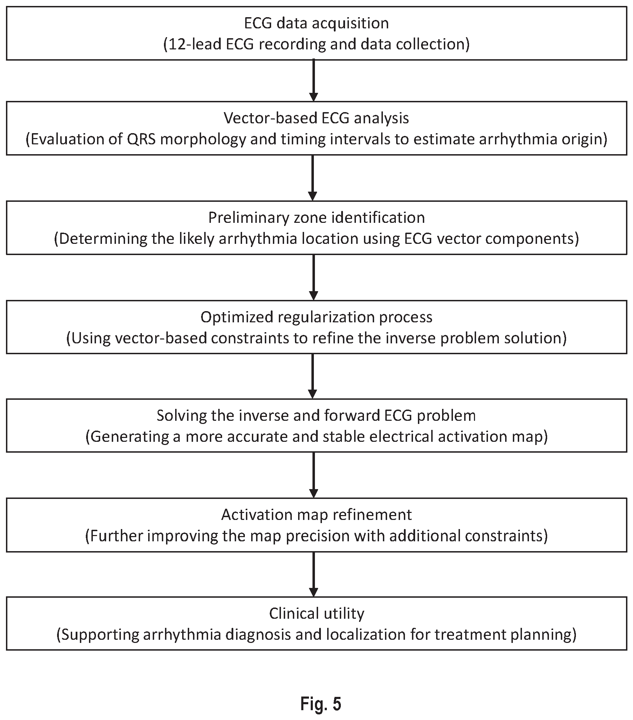

Figure 5 shows a flowchart of a method for generating a cardiac activation map. The method comprises ECG data acquisition which can be 12-lead ECG recording and data collection and further a vector-based ECG analysis which comprises evaluation of QRS morphology and timing intervals to estimate arrhythmia origin. The method further comprises preliminary zone identification which comprises determining the likely arrhythmia location using ECG vector components. The method further comprises an optimized regularization process using vector-based constraints to refine the inverse problem solution.

The method further comprises solving the inverse and forward ECG problem and thus generating a more accurate and stable electrical activation map. The method further comprises activation map refinement, further improving the map precision with additional constraints. The method further comprises supporting arrhythmia diagnosis and localization for treatment planning by a clinical utility.

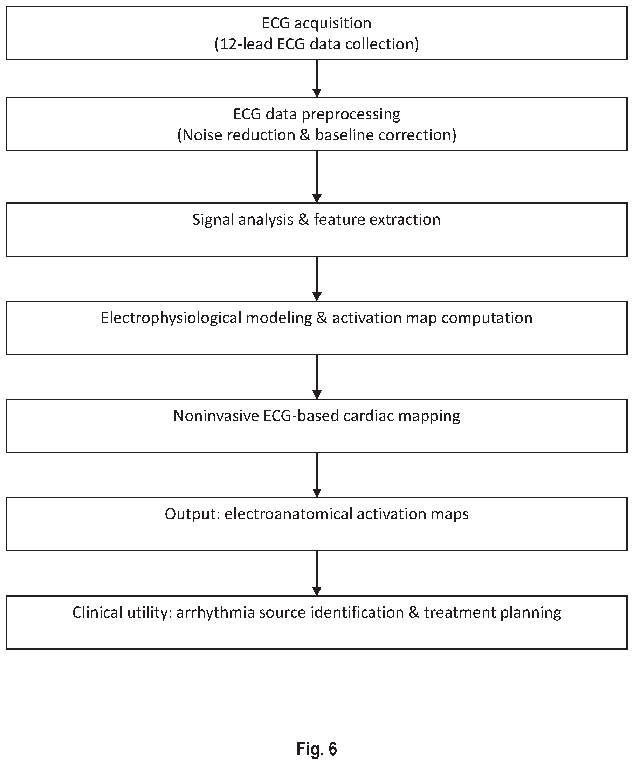

Figure 6 shows a flowchart of a method for non-invasive cardiac mapping. The method comprises ECG acquisition which can be 12-lead ECG data collection and ECG data preprocessing comprising noise reduction and baseline correction. The method further comprises signal analysis and feature extraction and electrophysiological modeling and activation map computation. The method further comprises non-invasive ECG-based cardiac mapping and outputting electroanatomical activation maps. The method further comprises arrhythmia source identification and treatment planning by a clinical utility.

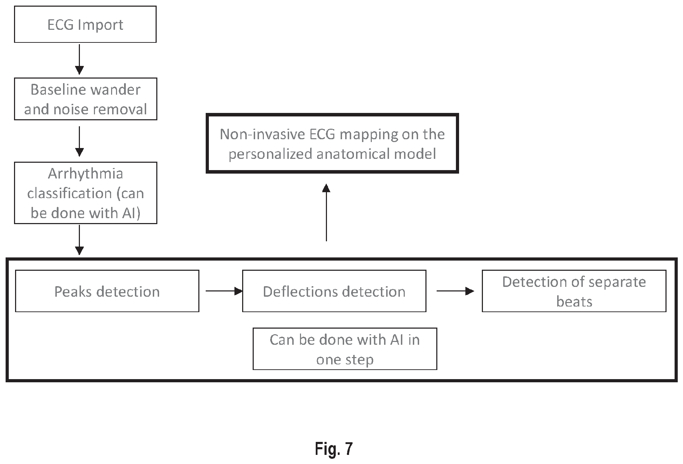

Figure 7 shows a flowchart of a method for analyzing ECG signals for use in non-invasive cardiac mapping. The method comprises importing ECG data and preprocessing the ECG data by removing baseline wander and noise. The method further comprises detecting peaks, deflections and separate beats.

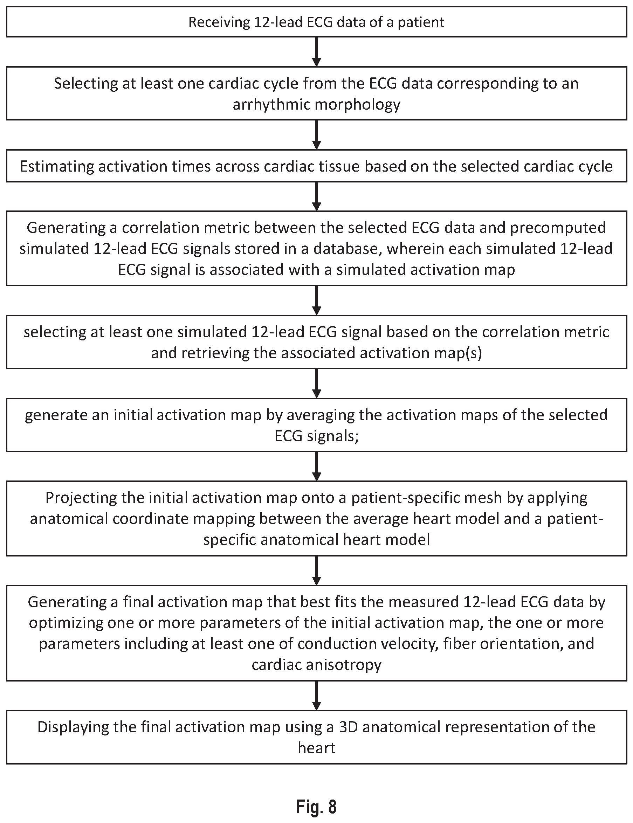

Figure 8 shows a flowchart of a method for generating a three-dimensional activation map of cardiac tissue based on a 12-lead electrocardiogram (ECG). The method comprises the step of receiving 12-lead ECG data 30 of a patient 1, the step of selecting at least one cardiac cycle 68 from the ECG data 30 corresponding to an arrhythmic morphology, the step of estimating activation times across cardiac tissue based on the selected cardiac cycle 68, and the step of generating a correlation metric between the selected ECG data 30 and precomputed simulated 12-lead ECG signals stored in a database 80, wherein each simulated 12-lead ECG signal is associated with a simulated activation map 67. The method further comprises selecting at least one simulated 12-lead ECG signal based on the correlation metric and retrieving the associated activation map(s) 67, generating an initial activation map 63 by averaging the activation maps 67 of the selected ECG signals, projecting the initial activation map 63 onto a patient-specific mesh by applying anatomical coordinate mapping between the average heart model and a patient-specific anatomical heart model, the step of generating a final activation map 65 that best fits the measured 12-lead ECG data by optimizing one or more parameters of the initial activation map 63, the one or more parameters including at least one of conduction velocity, fiber orientation, and cardiac anisotropy, and the step of displaying the final activation map 65 using a 3D anatomical representation of the heart.

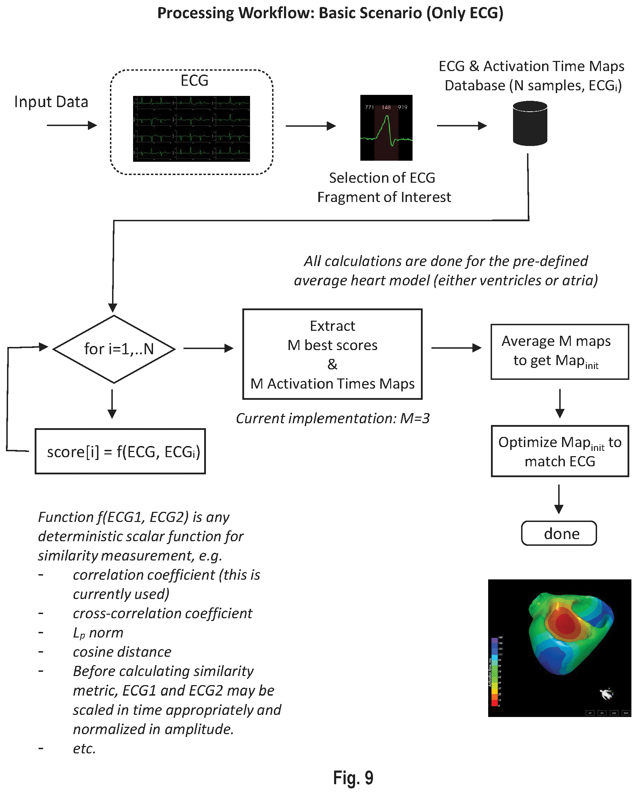

Figure 9 shows a flowchart of a further method for generating a three-dimensional activation map of cardiac tissue based on a 12-lead electrocardiogram (ECG). Input data from a patient 1 is acquired using an ECG machine 10, resulting in ECG data 30. From the ECG data 30, a user 2 selects an ECG fragment 66 comprising at least one cardiac cycle 68 that corresponds to an arrhythmic morphology. The selection of the ECG fragment 66 can also be performed automatically without any interaction with the user 2. The selected ECG fragment 66 is compared against a database 80 containing precomputed simulated 12-lead ECG signals, each associated with a simulated activation map 67.

A loop iterates over the simulated ECG data in the database 80. For each simulated ECG signal, a correlation metric is calculated to determine its similarity to the selected ECG fragment 66. The metric function may include various similarity measures such as correlation coefficient, cross-correlation, Lₚ norm, or cosine distance.

After computing the similarity scores, a subset of simulated ECG signals with the highest scores is selected. The corresponding simulated activation maps 67 for these signals are retrieved from the database 80. The current implementation selects M=3 maps.

These selected activation maps 67 are averaged to generate an initial activation map 63. This initial activation map 63 is further optimized to match the measured ECG data 30 by adjusting one or more parameters, such as conduction velocity, fiber orientation, or cardiac anisotropy.

The result is a final activation map 65. The final activation map 65 is displayed using a three-dimensional anatomical representation of the heart 16.

Figure 10 shows a flowchart of a further method for generating a three-dimensional activation map of cardiac tissue based on a 12-lead electrocardiogram (ECG). The method comprises the step of receiving 12-lead ECG data 30 of a patient 1, the step of selecting at least one cardiac cycle 68 from the ECG data 30 corresponding to an arrhythmic morphology, the step of estimating activation times across cardiac tissue based on the selected cardiac cycle 68, and the step of generating a correlation metric between the selected ECG data 30 and precomputed simulated 12-lead ECG signals stored in a database 80, wherein each simulated 12-lead ECG signal is associated with a simulated activation map 67. The method further comprises selecting at least one simulated 12-lead ECG signal based on the correlation metric and retrieving the associated activation map(s) 67, generating an initial activation map 63 by averaging the activation maps 67 of the selected ECG signals, projecting the initial activation map 63 onto a patient-specific mesh by applying anatomical coordinate mapping between the average heart model and a patient-specific anatomical heart model, the step of generating a final activation map 65 that best fits the measured 12-lead ECG data by optimizing one or more parameters of the initial activation map 63, the one or more parameters including at least one of conduction velocity, fiber orientation, and cardiac anisotropy, and the step of displaying the final activation map 65 using a 3D anatomical representation of the heart.

After computing the similarity scores, a subset of simulated ECG signals with the highest scores is selected. The corresponding simulated activation maps 67 for these signals are retrieved from the database 80. The current implementation selects M=3 maps.

These selected activation maps 67 are averaged to generate an initial activation map 63.

This initial activation map 63 is further optimized to match the measured ECG data 30 by adjusting one or more parameters, such as conduction velocity, fiber orientation, or cardiac anisotropy.

The result is a final activation map 65. The final activation map 65 is displayed using a three-dimensional anatomical representation of the heart 16.

First itemized list:
**1.** A computer-implemented method for generating a cardiac activation map, the method comprising:
   receiving 12-lead ECG data of a patient;
   performing vector-based analysis on the ECG data to identify an approximate region of origin of an arrhythmia, wherein the analysis is based on QRS morphology and timing intervals;
   generating the cardiac activation map by solving an inverse ECG problem, wherein solving the problem comprises a regularization procedure and wherein the identified approximate region of origin is used as a constraint in the regularization procedure; and
   outputting the cardiac activation map for clinical interpretation.
**2.** A computer-implemented method for generating a cardiac activation map, the method comprising:
   receiving ECG data of a patient;
   identifying an approximate region of origin of an arrhythmia; and
   generating the cardiac activation map based on the identified approximate region.
**3.** The computer-implemented method of item 2, wherein ECG data is 12-lead ECG data.
**4.** The computer-implemented method of item 3, wherein the 12-lead ECG data is obtained during an arrhythmic episode.
**5.** The computer-implemented method of one of items 2-4, wherein identifying the approximate region of origin comprises performing a vector-based analysis on the ECG data.
**6.** The computer-implemented method of item 5, wherein the vector-based analysis is based on QRS morphology and/or timing intervals.
**7.** The computer-implemented method of one of items 2-6, further comprising outputting the cardiac activation map.
**8.** A system for generating a cardiac activation map, the system comprising:
   an input interface configured to receive ECG data;
   a processor configured to:
      identify an approximate region of origin of an arrhythmia; and
      generate the cardiac activation map based on the identified approximate region; and
   an output interface for outputting the activation map to a user.
**9.** A computer program comprising instructions which, when the program is executed by a computer, cause the computer to carry out the method of any one of items 1-7.
**10.** A computer-readable medium comprising instructions which, when executed by a computer, cause the computer to carry out the method of any one of items 1-7.
**11.** A data processing apparatus, comprising means for carrying out the method of items 1-7.

The items of the first itemized list relate to a method for generating robust electrical activation maps of the heart by solving inverse electrocardiographic (ECG) problems using vector-based regularization derived from 12-lead ECG data. The method relates to a process that utilizes vector-based ECG information to optimize the computational resolution of inverse problems in the context of cardiac mapping.

Traditionally, the inverse ECG problem-which involves calculating an internal cardiac activation map based on non-invasive surface measurements such as those from a 12-lead ECG-has been characterized as mathematically ill-posed. This means that small measurement errors can lead to large inaccuracies in the calculated map, particularly when physiological or anatomical constraints are not available or are insufficiently defined. As a result, regularization methods used in the solution of the inverse problem often fail to yield reliable or clinically meaningful results.

The items introduce a novel approach to overcome these limitations by incorporating a vector-based analysis step prior to the solution of the inverse problem. The method applies well-established principles of ECG vector theory to the 12-lead ECG data to determine the approximate region of origin of an arrhythmia. This analysis focuses in particular on interpreting QRS morphology and timing intervals, allowing the system to infer the spatial zone of the arrhythmic source. The resulting information is then used as a constraint within the regularization step of the inverse problem.

By integrating this vector-based localization into the computational pipeline, the inverse problem is transformed from an under-determined system into one that is more tractable and bounded by clinically relevant parameters. The constrained regularization procedure thereby benefits from improved robustness and convergence properties, leading to enhanced accuracy and precision in the generation of activation maps.

The technical implementation comprises three aspects: (1) the integration of 12-lead ECG vector theory into the analysis process to derive a physiological estimation of arrhythmia origin, (2) the use of this estimation to guide and constrain the regularization procedure when solving the inverse problem, and (3) the generation of cardiac activation maps with improved spatial resolution and clinical reliability.

This method addresses the inherent instability of ill-posed inverse ECG problems by narrowing the solution space using physiologically meaningful constraints. It offers a non-invasive, cost-effective, and efficient alternative to catheter-based invasive mapping procedures, thereby reducing patient risk and resource burden. It supports clinical workflows by enabling faster identification of arrhythmic zones, which streamlines diagnosis and therapeutic planning. Furthermore, it introduces a data-driven enhancement to standard regularization techniques, moving beyond generic mathematical assumptions toward patient-specific, physiology-informed constraints. The method exhibits broad applicability across different types of arrhythmias and patient anatomies, making it a versatile and scalable tool for diverse clinical environments. By enabling the creation of more reliable and accurate electrical activation maps from standard surface ECG data, the method improves the clinical utility of non-invasive cardiac mapping.

The second itemized list refers to the aspect relating to a computer-implemented method for non-invasive cardiac mapping. The items of the second itemized list can be combined with one or more items of all other itemized lists in this document as well as with one or more features of the claims.

Second itemized list:
**1.** A computer-implemented method for non-invasive cardiac mapping, the method comprising:
   receiving 12-lead ECG data of a patient;
   preprocessing the ECG data comprising noise reduction and baseline correction;
   selecting an average heart model from a set of pre-defined anatomical models based on parameters of the patient including gender and estimated heart position within the torso;
   generating an electroanatomical activation map of the heart by processing the ECG data using the selected heart model and an electrophysiological simulation;
   identifying a region of origin and propagation pathways of the arrhythmia based on the generated activation map; and
   outputting the electroanatomical activation map, the identified region of origin and the identified propagation pathways for clinical interpretation.
**2.** A computer-implemented method for generating a cardiac activation map, the method comprising:
   receiving ECG data of a patient;
   generating an electroanatomical activation map of the heart by processing the ECG data using a heart model and an electrophysiological simulation; and
   identifying a region of origin and/or propagation pathways of the arrhythmia based on the generated activation map.
**3.** The computer-implemented method of item 2, wherein the ECG data is 12-lead ECG data.
**4.** The computer-implemented method of item 3, wherein the 12-lead ECG data is obtained during an arrhythmic episode.
**5.** The computer-implemented method of item 4, wherein only 12-lead ECG data is used as input data for generating the electroanatomical activation map.
**6.** The computer-implemented method of items 2-5, further comprising preprocessing the ECG data.
**7.** The computer-implemented method of item 6, wherein the preprocessing comprises noise reduction and/or baseline correction.
**8.** The computer-implemented method of items 2-7, further comprising selecting an average heart model from a set of pre-defined anatomical models, wherein the selected model is used for generating the electroanatomical activation map.
**9.** The computer-implemented method of item 8, wherein selecting the heart model is based on parameters of the patient including gender and/or estimated heart position within the torso.
**10.**The computer-implemented method of items 8 or 9, wherein the heart model is selected from a database containing gender-specific anatomical variations.
**11.**A system for non-invasive cardiac mapping, comprising:
   an input interface configured to receive ECG data;
   a processor configured to:
      generate an electroanatomical activation map of the heart by processing the ECG data using a heart model and an electrophysiological simulation; and
      identify a region of origin and/or propagation pathways of the arrhythmia based on the generated activation map; and
   an output interface for outputting the activation map to a user.
**12.**A computer program comprising instructions which, when the program is executed by a computer, cause the computer to carry out the method of any one of items 1-10.
**13.**A computer-readable medium comprising instructions which, when executed by a computer, cause the computer to carry out the method of any one of items 1-10.
**14.**A data processing apparatus, comprising means for carrying out the method of items 1-10.

The items of the second itemized list concern a method for non-invasive cardiac mapping that computes electroanatomical activation maps of cardiac arrhythmias using only data derived from standard 12-lead electrocardiograms (ECG), without requiring imaging modalities such as computed tomography (CT) or magnetic resonance imaging (MRI). This method is implemented through a novel algorithm that integrates electrophysiological modeling with advanced signal processing and computational techniques. It is designed to provide physicians with a rapid, accurate, and cost-effective diagnostic tool for identifying the origins and propagation pathways of cardiac arrhythmias, including both focal and reentrant types.

A feature of the method is its tomography-free approach. Unlike conventional mapping techniques that require imaging data to reconstruct individualized anatomical models, the invention employs an average heart model as the basis for its computations. This strategy eliminates or reduces the dependence on costly and resource-intensive imaging systems, making the method widely deployable, particularly in settings where advanced imaging tools are not available. The algorithm further enhances data interpretation by incorporating individualized parameters such as the patient's gender and the anatomical position of the heart within the torso, which contributes to the accuracy and personalization of the mapping output.

The method yields electroanatomical activation maps that visually represent the spatial and temporal characteristics of electrical signal propagation in the heart. These outputs are specifically designed to assist clinicians in diagnosing arrhythmia sources and understanding their behavior, thereby facilitating informed and timely therapeutic decisions. By presenting the results in a user-friendly format, the algorithm supports swift clinical evaluation without requiring additional interpretation infrastructure.

The method addresses several challenges in the field of cardiac electrophysiology. It enhances accessibility by relying exclusively on ECG systems, which are widely available even in resource-limited healthcare environments. It reduces the cost associated with arrhythmia diagnostics and treatment by removing the need for expensive imaging procedures. It delivers results rapidly, promoting efficient clinical workflows. The method achieves a high level of precision through the integration of gender-specific physiological characteristics and spatial modeling of the heart's position, leading to reliable and reproducible diagnostic outcomes. The method contributes to improved clinical outcomes by equipping physicians with detailed visualizations of arrhythmia mechanisms, thus supporting more targeted and effective treatment strategies.

The third itemized list refers to a method for generating a three-dimensional activation map of cardiac tissue based on a 12-lead electrocardiogram (ECG). The items of the third itemized list can be combined with one or more items of all other itemized lists in this document as well as with one or more features of the claims.

Third itemized list:
1. A computer-implemented method for generating a three-dimensional activation map of cardiac tissue based on a 12-lead electrocardiogram (ECG), the method comprising:
   receiving 12-lead ECG data of a patient;
   selecting at least one cardiac cycle from the ECG data corresponding to an arrhythmic morphology;
   estimating activation times across cardiac tissue based on the selected cardiac cycle;
   generating a correlation metric between the selected ECG data and precomputed simulated 12-lead ECG signals a database, wherein each simulated 12-lead ECG signal is associated with a simulated activation map;
   selecting at least one simulated 12-lead ECG signal based on the correlation metric and retrieving the associated activation map(s);
   generate an initial activation map by averaging the activation maps of the selected ECG signals;
   generating a final activation map that best fits the measured 12-lead ECG data by optimizing one or more parameters of the initial activation map, the one or more parameters including at least one of conduction velocity, fiber orientation, and cardiac anisotropy; and
   displaying the final activation map using a 3D anatomical representation of the heart.
2. The computer-implemented method of item 1, further comprising projecting the initial activation map onto a patient-specific mesh by applying anatomical coordinate mapping between the average heart model and a patient-specific anatomical heart model.
3. A computer-implemented method for generating a cardiac activation map, the method comprising:
   receiving data for at least one cardiac cycle obtained from ECG data of a patient;
   determining at least one parameter representing cardiac activation based on the received data;
   obtaining from a database at least one activation map, wherein the obtained at least one activation map is selected from the database based on the determined at least one parameter;
   generating an initial activation map based on the at least one activation map obtained from the database; and
   generating a final activation map by optimizing the initial activation map.
4. The computer-implemented method of item 3, further comprising receiving ECG data of the patient.
5. The computer-implemented method of item 3 or 4, wherein the ECG data is 12-lead ECG data.
6. The computer-implemented method of item 4 or 5, further comprising selecting the at least one cardiac cycle from the ECG data.
7. The computer-implemented method of item 7, wherein the at least one cardiac cycle is selected corresponding to an arrhythmic morphology.
8. The computer-implemented method of one of items 3-7, wherein the at least one parameter representing cardiac activation is activation times across cardiac tissue.
9. The computer-implemented method of one of items 3-8, wherein obtaining the at least one activation map from the database comprises comparing the selected at least one cardiac cycle to ECG data stored in the database.
10.The computer-implemented method of item 9, wherein the ECG data stored in the database comprises precomputed simulated ECG signals.
11.The computer-implemented method of one of items 9 and 10, wherein the step of comparing comprises generating a correlation metric between the selected at least one cardiac cycle and at least one of the precomputed simulated ECG signals.
12.The computer-implemented method of item 11, wherein the step of comparing further comprises selecting at least one precomputed simulated ECG signal based on the correlation metric and obtain the at least one activation map which is associated with the selected at least one precomputed simulated ECG signal.
13.The computer-implemented method of one of items 3-12, wherein generating the initial activation map comprises averaging the at least one obtained activation maps.
14.The computer-implemented method of one of items 3-13, wherein generating the final activation map comprises optimizing one or more parameters of the initial activation map.
15. The computer-implemented method of one of item 14, wherein the one or more parameters include at least one of conduction velocity, fiber orientation, and cardiac anisotropy.
16.The computer-implemented method of one of items 3-15, further comprising outputting the activation map to a user.
17.The computer-implemented method of one of items 3-15, further comprising projecting the initial activation map onto a patient-specific model of the heart.
18.A system for generating a cardiac activation map, comprising:
   an input interface configured to receive data for at least one cardiac cycle obtained from ECG data of a patient;
   a database configured for storing at least one activation map;
   a processor configured to:
      determine at least one parameter representing cardiac activation based on the received data;
      obtain from the database at least one activation map, wherein the obtained at least one activation map is selected from the database based on the determined at least one parameter;
      generate an initial activation map based on the at least one activation map obtained from the database; and
      generate a final activation map by optimizing the initial activation map.
19.The system of item 18, further comprising an output interface for outputting the activation map to a user.
20.A computer program comprising instructions which, when the program is executed by a computer, cause the computer to carry out the method of any one of items 1-17.
21.A computer-readable medium comprising instructions which, when executed by a computer, cause the computer to carry out the method of any one of items 1-17.
22.A data processing apparatus, comprising means for carrying out the method of items 1-17.

The fourth itemized list refers to method for generating a dataset of simulated cardiac activation patterns. The items of the fourth itemized list can be combined with one or more items of all other itemized lists in this document as well as with one or more features of the claims.

Fourth itemized list:
1. A computer-implemented method for generating a dataset of simulated cardiac activation patterns and corresponding 12-lead ECG signals, the method comprising:
   providing a three-dimensional anatomical model of the heart and torso, wherein the heart is represented by a tetrahedral mesh incorporating myocardial fiber orientations and tissue conductivities;
   defining a set of simulation parameters comprising activation source locations and conduction velocity tensors;
   simulating cardiac activation by solving an anisotropic eikonal equation on the mesh to compute activation times across the myocardium, using a focal excitation at a defined source location;
   modulating conduction velocities in accordance with the defined tensors to reflect anisotropic propagation, including pathological regions with reduced conduction velocity to mimic scar or fibrotic tissue;
   repeating simulating cardiac activation for multiple variations of the simulation parameters according to a quasi-random Halton sampling scheme;
   for each simulation, computing corresponding synthetic 12-lead ECG signals using a lead field method applied to the calculated activation data;
   storing, for each simulation, the activation map, the associated simulated ECG signals, and metadata describing the simulation parameters.
2. A computer-implemented method for generating a dataset of activation maps and corresponding ECG signals, the method comprising:
   generating a first cardiac activation map based on an electrical activation model for a first set of simulation parameters;
   generating a second cardiac activation map based on an electrical activation model for a second set of simulation parameters, wherein the first set is different from the second set;
   generating first ECG signals corresponding to the first cardiac activation map; and
   generating second ECG signals corresponding to the second cardiac activation map.
3. The computer-implemented method of item 2, further comprising storing the first cardiac activation map, the second cardiac activation map, the first ECG signals, the second ECG signals, the first set of simulation parameters and the second set of simulation parameters in a database.
4. A system for generating a dataset of activation maps and corresponding ECG signals, comprising:
   a database configured for storing at the activation maps, the corresponding ECG signals and the corresponding sets of simulation parameters; and
   a processor configured to:
      generate a first cardiac activation map based on an electrical activation model for a first set of simulation parameters;
      generate a second cardiac activation map based on an electrical activation model for a second set of simulation parameters, wherein the first set is different from the second set;
      generate first ECG signals corresponding to the first cardiac activation map; and
      generate second ECG signals corresponding to the second cardiac activation map.
5. A computer program comprising instructions which, when the program is executed by a computer, cause the computer to carry out the method of any one of items 1-3.
6. A computer-readable medium comprising instructions which, when executed by a computer, cause the computer to carry out the method of any one of items 1-3.
7. A data processing apparatus, comprising means for carrying out the method of items 1-3.

The fifth itemized list refers to a computer-implemented method and corresponding system for generating a three-dimensional electroanatomical activation map of cardiac tissue based on standard 12-lead electrocardiogram (ECG) data. The items of the fifth itemized list can be combined with one or more items of the preceding itemized lists as well as with one or more features of the claims.

The fifth itemized list concerns non-invasive 12-lead ECG interpretation without other signals and the generation of a three-dimensional electroanatomical activation map based on electrical activation information determined from the 12-lead ECG data. The method comprises computing activation times across cardiac tissue using an electrophysiological model and generating a three-dimensional representation of cardiac electrical activity.

Fifth itemizes list:
1. A computer-implemented method for generating a three-dimensional electroanatomical activation map of a heart of a patient, the method comprising:
   receiving standard 12-lead electrocardiogram (ECG) data of the patient;
   interpreting the 12-lead ECG data to determine electrical activation information of cardiac tissue without other signals;
   computing electrical activation times across cardiac tissue by solving an inverse electrocardiographic problem that relates body-surface ECG measurements to cardiac electrical sources using an electrophysiological model;
   generating a three-dimensional electroanatomical activation map of at least one cardiac chamber based on the computed activation times; and
   outputting the electroanatomical activation map for visualization.
2. The method of item 1, further comprising preprocessing the ECG data, including at least one of noise reduction and baseline correction.
3. The method of item 1 or 2, further comprising selecting at least one cardiac cycle corresponding to an arrhythmic morphology.
4. The method of any one of items 1-3, wherein solving the inverse electrocardiographic problem comprises using a finite element solver to model a relationship between cardiac electrical sources and ECG signals measured on a body surface.
5. The method of any one of items 1-4, wherein the electrophysiological model comprises solving an anisotropic eikonal equation to simulate propagation of electrical excitation through myocardial tissue.
6. 6. The method of any one of items 1-5, wherein the heart is represented by a three-dimensional anatomical model comprising a tetrahedral mesh including myocardial fiber orientation information.
7. The method of any one of items 1-6, wherein generating the electroanatomical activation map comprises generating at least one of:
   an activation time map,
   a propagation map,
   a phase map.
8. The method of any one of items 1-7, wherein the electroanatomical activation map represents atrial and ventricular chambers in a panoramic manner.
9. A system for generating a three-dimensional electroanatomical activation map of a heart of a patient, comprising:
   an ECG machine (10) configured to acquire standard 12-lead ECG data (30) from a patient (1);
   a data integration hub computer (14) comprising a processor (72) and a memory;
   a 3D modeling software (17) stored in the memory and executable by the processor (72), the software comprising:
      an ECG analysis module (21) configured to interpret the 12-lead ECG data without other signals to determine electrical activation information of cardiac tissue; and
      a maps module (25) configured to compute electrical activation times by solving an inverse electrocardiographic problem using an electrophysiological model and to generate a three-dimensional electroanatomical activation map (65); and
      a computer screen (15) configured to display the electroanatomical activation map (65).
10. The system of item 9, wherein the electrophysiological model comprises a finite element solver configured to model a relationship between cardiac electrical sources and ECG signals measured on a body surface.
11. The system of item 9 or 10, wherein the heart is represented by a three-dimensional anatomical model comprising a tetrahedral mesh including myocardial fiber orientation information.
12. A system for generating a three-dimensional electroanatomical activation map of a heart of a patient, comprising:
   means for receiving standard 12-lead electrocardiogram (ECG) data of the patient; means for interpreting the 12-lead ECG data without other signals to determine electrical activation information of cardiac tissue;
   means for solving an inverse electrocardiographic problem using an electrophysiological model to compute electrical activation times across cardiac tissue; and
   means for generating and displaying a three-dimensional electroanatomical activation map based on the computed activation times.
13. A system for non-invasive cardiac mapping, comprising:
   an input interface configured to receive standard 12-lead electrocardiogram (ECG) data of a patient;
   processing circuitry configured to interpret the 12-lead ECG data without other signals and to determine electrical activation characteristics of cardiac tissue using an electrophysiological model; and
   an output interface configured to provide a three-dimensional representation of cardiac electrical activity.
14. A computer-implemented method for non-invasive cardiac mapping, comprising:
   receiving standard 12-lead electrocardiogram (ECG) data;
   interpreting the ECG data without other signals;
   determining electrical activation characteristics of cardiac tissue using a computational model; and
   generating a three-dimensional representation of cardiac electrical activity based on the determined characteristics.
15.One or more non-transitory computer-readable media storing instructions which, when executed by processing circuitry, cause the processing circuitry to perform operations comprising:
   receiving standard 12-lead ECG data;
   interpreting the ECG data without other signals;
   solving an inverse electrocardiographic problem using an electrophysiological model to compute electrical activation times; and
   generating a three-dimensional electroanatomical activation map.
16.A data processing apparatus comprising processing circuitry configured to perform the method of any one of items 1-8.

### DEFINITIONS

A "patient" designates a human being.

The verb to "personalize" (in the sense of personalize, personalise, individualize, individualise) can be replaced by: make personal or more personal

An "ECG machine" designates a device that is capable of receiving standard 12-lead ECG data from a patient and generating a standard 12-lead ECG.

An "ultrasound machine" designates any device that can be used to generate ultrasound images of the heart.

A "data integration hub computer" designates a computer that is capable of receiving data from multiple sources, and integrating that data into a single, unified view.

A "computer screen" designates a display device, such as a computer monitor, a tablet, a smartphone, or a head-mounted display.

A "3D heart model representation" designates a representation of the heart in 3D space.

A "3D modeling software" designates software that can be used to create 3D models of the heart,.

An "ECG analysis module" designates the algorithms that process the ECG data to calculate the propagation of electrical excitation across the heart.

As used herein, the term "cardiovascular structures" refers to any structure in the heart that may be altered by electrical excitation.

As used herein, the term "invasive electrophysiological data" refers to any cardiac excitation that causes electrical activity in the heart.

As used herein, the term "heart models" refers to a collection of cardiac structures that are modelled using imaging data.

A "personalized 3D model" designates a model of the heart that is personalized to a particular patient.

As used herein, the term "panoramic electroanatomical maps" refers to a detailed representation of the heart including at least one of activation maps, propagation maps, etc.

A "heart model for healthcare professionals" designates a model of the heart that is personalized for a particular patient.

As used herein, the term "areas of fibrotic tissue" refers to areas of fibrous tissue that may be identified by imaging.

As used herein, the term "electrical excitation" refers to a propagation of electrical signals across the heart.

As used herein, the term "existing clinical workflows" refers to existing workflows that have a variety of functions, such as those associated with cardiac diagnostic analysis.

A "personalized 3D heart model" designates a model of the heart that is personalized to a particular patient, and the term personalized 3D heart model is used interchangeably with the term personalized 3D heart model.

A "healthcare professional" designates a physician, nurse, physician assistant, or other healthcare professional.

As used herein, the term "fibrotic tissue" refers to cardiac structures that have been damaged or deteriorated.

As used herein, the term "cardiovascular electrical sources" refers to any source of electrical energy that can be applied to a heart muscle.

A "cardiac diagnostic analysis" designates the analysis of the heart using the personalized 3D models.

A "standard 12-lead ECG datum" designates a set of 12-lead ECG data that has been recorded in a standard manner.

A "calculated excitation" designates the propagation of electrical excitation across the heart, which is calculated by the advanced algorithms.

An "activation map" designates an activation map with local timings of the electrical excitation across the heart.

A "propagation map" designates a map of the propagation of electrical excitation across the heart.

A "phase map" designates a map of the phase of the electrical excitation wavefronts in the heart.

A "rotor density map" designates a map of the number of rotors per unit volume of the heart.

A "cardiac procedure" designates any procedure that involves the heart.

An "impedance measurement" designates the measurement of the electrical impedance of the heart.

A "catheter position" designates the location of the catheter tip within the heart.

As used herein, the term "finite element solver" refers to a mathematical solution that uses two or more elements as boundary points.

A "body surface" designates the surface of the body that is in contact with the skin.

Protection may be sought for combinations of features which are disclosed in the referenced earlier patent applications DE102024114031.3 of 20 May 2024 and EP25177220.8 of 19 May 2025, the contents of which are herein incorporated by reference. It is disclosed there how these features combinations contribute to achieving the technical aim of the present application and they are thus comprised in the solution of the technical problem underlying the subject matter of the present application. The features and combinations which are disclosed in the reference documents implicitly belong to the description of the subject matter in the present application and thus to the content of the present application as filed.

### REFERENCE NUMERAL LIST

- 1: patient
- 2: user
- 10: ECG machine
- 11: MRI
- 12: CT machine
- 12: CT
- 12: imaging data including CT
- 13: ultrasound machine
- 13: ultrasound
- 14: data integration hub computer
- 15: computer screen
- 16: 3D heart model representation
- 16: heart
- 17: 3D modeling software
- 21: ECG analysis module
- 25: maps module
- 30: ECG data
- 32: set of ECG data
- 34: first set of ECG data
- 36: second set of ECG data
- 40: first electroanatomic map
- 42: second electroanatomic map
- 44: first quantitative metrics
- 46: second quantitative metrics
- 50: report
- 60: approximate region of origin
- 62: cardiac activation map
- 63: initial activation map
- 64: cardiac image
- 65: final activation map
- 66: ECG fragment
- 67: simulated activation map
- 68: cardiac cycle
- 70: input interface
- 72: processor
- 74: output interface
- 80: database

## Claims

1. A computer-implemented method for generating a three-dimensional activation map of cardiac tissue of a patient based on non-invasive 12-lead electrocardiogram (ECG) interpretation without other signals, the method comprising:
receiving 12-lead ECG data of a patient;
selecting at least one cardiac cycle from the ECG data corresponding to an arrhythmic morphology;
estimating activation times across cardiac tissue based on the selected cardiac cycle;
generating a correlation metric between the selected cardiac cycle and a plurality of precomputed simulated 12-lead ECG signals stored in a database, wherein each simulated 12-lead ECG signal is associated with a simulated activation map;
selecting at least one simulated 12-lead ECG signal based on the correlation metric and retrieving the associated activation map(s);
generating an initial activation map based on the at least one retrieved simulated activation map;
generating a final activation map that best fits the measured 12-lead ECG data by optimizing one or more parameters of the initial activation map, the one or more parameters including at least one of conduction velocity, fiber orientation, and cardiac anisotropy; and
displaying the final activation map using a 3D anatomical representation of the heart.

2. The method of claim 1, wherein selecting at least one simulated 12-lead ECG signal based on the correlation metric comprises selecting at least two simulated 12-lead ECG signals, retrieving the associated simulated activation map comprises retrieving at least two associated simulated activation maps, and generating the initial activation map comprises averaging the at least two retrieved simulated activation maps corresponding to the highest correlation metric values.

3. The method of claim 1 or 2, wherein the method is performed without acquiring patient-specific tomographic imaging data.

4. The method of any one of claims 1-3, wherein the method is performed without acquiring additional body-surface electrical signals other than the 12-lead ECG data.

5. The method of any one of claims 1-4, wherein generating the final activation map comprises solving an inverse electrocardiographic problem relating body-surface ECG measurements to cardiac electrical sources.

6. The method of claim 5, wherein solving the inverse electrocardiographic problem comprises using a finite element solver to model a relationship between cardiac electrical sources and ECG signals measured on a body surface.

7. The method of any one of claims 1-6, further comprising selecting a heart model from a plurality of pre-defined anatomical heart models stored in a database.

8. The method of claim 7, wherein the heart model is selected based on at least one patient-specific parameter including gender and estimated heart position within a torso.

9. The method of claim 7 or 8, wherein the selected heart model comprises a three-dimensional anatomical representation including a tetrahedral mesh.

10. The method of any one of claims 7-9, wherein the final activation map is generated using the selected heart model without requiring patient-specific imaging data.

11. The method of any one of claims 1-10, wherein the precomputed simulated 12-lead ECG signals stored in the database are generated by:
solving an anisotropic eikonal equation on a three-dimensional heart model to compute activation times; and
computing corresponding 12-lead ECG signals using a lead field method.

12. The method of claim 11, wherein activation source locations and conduction velocity tensors are varied across multiple simulations.

13. The method of claim 11 or 12, wherein the simulations are repeated according to a quasi-random sampling scheme.

14. The method of any one of claims 11-13, wherein for each simulation the database stores:
the activation map,
the corresponding simulated 12-lead ECG signals, and
metadata describing simulation parameters.

15. The method of any one of claims 1-14, wherein the correlation metric comprises a Spearman correlation coefficient.

16. The method of any one of claims 1-15, wherein optimizing the electrophysiological parameters comprises adjusting at least one of:
regional conduction velocity,
myocardial fiber orientation, and
cardiac anisotropy.

17. A system for generating a three-dimensional electroanatomical activation map of a heart of a patient based on non-invasive 12-lead electrocardiogram (ECG) interpretation without other signals, comprising:
an input interface configured to receive standard 12-lead ECG data of the patient;
a database configured for storing a plurality of precomputed simulated 12-lead ECG signals, each simulated 12-lead ECG signal being associated with at least one simulated activation map;
a processor configured to:
select at least one cardiac cycle from the ECG data corresponding to an arrhythmic morphology;
estimate activation times across cardiac tissue based on the selected cardiac cycle;
generate a correlation metric between the selected cardiac cycle and the simulated 12-lead ECG signals stored in the database;
select at least one simulated 12-lead ECG signal based on the correlation metric and retrieve the associated simulated activation map from the database;
generate an initial activation map based on the retrieved simulated activation map obtained from the database; and
generate a final activation map by optimizing one or more electrophysiological parameters of the initial activation map, the parameters including at least one of conduction velocity, fiber orientation, and cardiac anisotropy; and
an output interface configured to display the final activation map using a three-dimensional anatomical representation of the heart.

18. The system of claim 17, wherein the processing circuitry is configured to select at least two simulated 12-lead ECG signals based on the correlation metric, retrieve at least two associated simulated activation maps, and generate the initial activation map by averaging the at least two retrieved simulated activation maps corresponding to the highest correlation metric values.

19. The system of claim 17 or 18, wherein the system is configured to operate without acquiring patient-specific tomographic imaging data.

20. The system of any one of claims 17-19, wherein the system is configured to operate without acquiring additional body-surface electrical signals other than the 12-lead ECG data.

21. The system of any one of claims 17-20, wherein the processing circuitry is configured to solve an inverse electrocardiographic problem relating body-surface ECG measurements to cardiac electrical sources.

22. The system of claim 21, wherein the processing circuitry comprises or is configured to use a finite element solver to model a relationship between cardiac electrical sources and ECG signals measured on a body surface.

23. The system of any one of claims 17-22, wherein the processing circuitry is further configured to select a heart model from a plurality of pre-defined anatomical heart models stored in a database.

24. The system of claim 23, wherein the heart model is selected based on at least one patient-specific parameter including gender and estimated heart position within a torso.

25. The system of claim 23 or 24, wherein the selected heart model comprises a three-dimensional anatomical representation including a tetrahedral mesh.

26. The system of any one of claims 17-25, wherein the simulated 12-lead ECG signals stored in the database are generated by solving an anisotropic eikonal equation on a three-dimensional heart model to compute activation times and computing corresponding 12-lead ECG signals using a lead field method.

27. The system of claim 26, wherein activation source locations and conduction velocity tensors are varied across multiple simulations.

28. The system of claim 26 or 27, wherein for each simulation the database stores the activation map, the corresponding simulated 12-lead ECG signals, and metadata describing simulation parameters.

29. A computer program comprising instructions which, when the program is executed by a computer, cause the computer to carry out the method of any one of claims 1-16.

30. A computer-readable medium comprising instructions which, when executed by a computer, cause the computer to carry out the method of any one of claims 1-16.

31. A data processing apparatus, comprising means for carrying out the method of claims 1-16.
